# EUROPEAN PATENT SPECIFICATION

(11) **EP 3 097 910 B1**
(45) Date of publication and mention of the grant of the patent: **13.05.2020**
(21) Application number: 15740348.6
(22) Date of filing: 23.01.2015
(51) Int. Cl.: A61K 31/20, A61K 31/201, A61K 31/202, A61P 29/00, C12N 9/88

(54) **ANTI-INFLAMMATORY AGENT CONTAINING RARE FATTY ACID**
ENTZÜNDUNGSHEMMENDES MITTEL MIT SELTENER FETTSÄURE
AGENT ANTI-INFLAMMATOIRE CONTENANT UN ACIDE GRAS RARE

(30) Priority: 24.01.2014 JP 2014011866; 08.08.2014 JP 2014162982
(43) Date of publication of application: 30.11.2016
(73) Proprietor: Kyoto University, Kyoto-shi, Kyoto 606-8501 (JP); Nitto Pharmaceutical Industries, Ltd., Muko-shi, Kyoto 617-0006 (JP)
(72) Inventor: OGAWA, Jun, Kyoto-shi Kyoto 606-8501 (JP); KISHINO, Shigenobu, Kyoto-shi Kyoto 606-8501 (JP); KAWADA, Teruo, Kyoto-shi Kyoto 606-8501 (JP); TAKAHASHI, Nobuyuki, Kyoto-shi Kyoto 606-8501 (JP); GOTO, Tsuyoshi, Kyoto-shi Kyoto 606-8501 (JP); SUGAWARA, Tatsuya, Kyoto-shi Kyoto 606-8501 (JP); YONEJIMA, Yasunori, Muko-shi Kyoto 617-0006 (JP)
(74) Representative: J A Kemp LLP
(86) International application number: PCT/JP2015/051844
(87) International publication number: WO 2015/111701

(56) References cited:
- WO-A1-99/16435
- WO-A1-2013/168310
- JP-A- 2009 051 732
- JP-A- 2009 545 527
- JP-A- 2011 184 411
- CELME VIEIRA ET AL: "Effect of ricinoleic acid in acute and subchronic experimental models of inflammation", MEDIATORS OF INFLAMMATION., vol. 9, no. 5, 1 January 2000 (2000-01-01) , pages 223-228, XP55414402, GB ISSN: 0962-9351, DOI: 10.1080/09629350020025737
- BODDU SAI H S ET AL: "Anti-inflammatory effects of a novel ricinoleic acid poloxamer gel system for transdermal delivery", INTERNATIONAL JOURNAL OF PHARMACEUTICS, ELSEVIER, AMSTERDAM, NL, vol. 479, no. 1, 24 December 2014 (2014-12-24), pages 207-211, XP029133048, ISSN: 0378-5173, DOI: 10.1016/J.IJPHARM.2014.12.051
- MURAKAMI ET AL: "New class of linoleic acid metabolites biosynthesized by corn and rice lipoxygenases: Suppression of proinflammatory mediator expression via attenuation of MAPK- and Akt-, but not PPAR@c-, dependent pathways in stimulated macrophages", BIOCHEMICAL PHARMACO, ELSEVIER, US, vol. 70, no. 9, 1 November 2005 (2005-11-01), pages 1330-1342, XP005092611, ISSN: 0006-2952, DOI: 10.1016/J.BCP.2005.07.032
- KAORI TANABE ET AL.: 'Nyusankin o Mochiita Unique na Hydroxy Shibosan Seisan ni Tsuite' JAPAN SOCIETY FOR BIOSCIENCE, BIOTECHNOLOGY, AND AGROCHEMISTRY TAIKAI KOEN YOSHISHU vol. 2007, 2007, page 45, XP008184361
- CIPOLLINA CHIARA ET AL.: 'Cyclooxygenase-2 generates anti-inflammatory omega-3 fatty acid derivatives in activated macrophages' FREE RADICAL BIOLOGY AND MEDICINE vol. 47, no. 1, 2009, page S121, XP055214926

### SEQUENCE LISTING

<110> Kyoto University
   NITTO PHARMACEUTICAL INDUSTRIES, LTD.
<120> Anti-inflammatory agent comprising rare fatty acids
<130> 092264
<150> JP 2014-011866
   <151> 2014-01-24
<150> JP 2014-162982
   <151> 2014-08-08
<160> 2
<170> PatentIn version 3.5
<210> 1
   <211> 1773
   <212> DNA
   <213> Lactobacillus acidophilus
<220>
   <221> CDS
   <222> (1)..(1773)
<400> 1
   atg cat tat agt agt ggt aat tat gaa gct ttt gta aac gca agt aaa          48
   Met His Tyr Ser Ser Gly Asn Tyr Glu Ala Phe Val Asn Ala Ser Lys
   1               5                   10                  15
   cct aag gat gtc gat cag aag tcc gca tat ctt gtt ggt tca ggt ttg          96
   Pro Lys Asp Val Asp Gln Lys Ser Ala Tyr Leu Val Gly Ser Gly Leu
               20                  25                  30
   gca tcg ctt gct agt gct gta ttt tta att cgt gat ggt cac atg aag         144
   Ala Ser Leu Ala Ser Ala Val Phe Leu Ile Arg Asp Gly His Met Lys
           35                  40                  45
   ggt gat aga att cat atc ctt gaa gaa ttg agc ctt cca ggt ggt tca         192
   Gly Asp Arg Ile His Ile Leu Glu Glu Leu Ser Leu Pro Gly Gly Ser
       50                  55                  60
   atg gat ggg atc tat aat aag caa aaa gaa agc tac atc att cgt ggt         240
   Met Asp Gly Ile Tyr Asn Lys Gln Lys Glu Ser Tyr Ile Ile Arg Gly
   65                  70                  75                  80
   ggt cgt gaa atg gaa gcc cat ttt gaa tgc ttg tgg gac ttg ttt aga         288
   Gly Arg Glu Met Glu Ala His Phe Glu Cys Leu Trp Asp Leu Phe Arg
                   85                  90                  95
   tcg att cca tca gct gaa aat aaa gat gaa tcg gtc ctg gat gaa ttt         336
   Ser Ile Pro Ser Ala Glu Asn Lys Asp Glu Ser Val Leu Asp Glu Phe
               100                 105                 110
   tac cgt tta aat aga aaa gat cca agt ttc gca aag act cgt gtc att         384
   Tyr Arg Leu Asn Arg Lys Asp Pro Ser Phe Ala Lys Thr Arg Val Ile
           115                 120                 125
   gtt aac cgc gga cat gaa ctt cca act gac ggt caa tta ctt ctt act         432
   Val Asn Arg Gly His Glu Leu Pro Thr Asp Gly Gln Leu Leu Leu Thr
       130                 135                 140
   ccc aag gct gtt aaa gaa att att gat ctt tgc tta act cct gaa aaa      480
   Pro Lys Ala Val Lys Glu Ile Ile Asp Leu Cys Leu Thr Pro Glu Lys
   145                 150                 155                 160
   gat tta caa aat aaa aaa att aat gaa gtc ttt agt aaa gaa ttt ttt      528
   Asp Leu Gln Asn Lys Lys Ile Asn Glu Val Phe Ser Lys Glu Phe Phe
                   165                 170                 175
   gaa tca aac ttc tgg ctt tac tgg tca acg atg ttt gcc ttt gag cca      576
   Glu Ser Asn Phe Trp Leu Tyr Trp Ser Thr Met Phe Ala Phe Glu Pro
               180                 185                 190
   tgg gca agt gcg atg gaa atg cgt cgt tac tta atg cgt ttt gtt caa      624
   Trp Ala Ser Ala Met Glu Met Arg Arg Tyr Leu Met Arg Phe Val Gln
           195                 200                 205
   cac gtt tct aca ctt aag aat tta tca tca cta cgc ttt act aag tat      672
   His Val Ser Thr Leu Lys Asn Leu Ser Ser Leu Arg Phe Thr Lys Tyr
       210                 215                 220
   aac caa tat gaa tca tta att tta cca atg gtt aaa tac ttg aaa gat      720
   Asn Gln Tyr Glu Ser Leu Ile Leu Pro Met Val Lys Tyr Leu Lys Asp
   225                 230                 235                 240
   cgc ggc gtg caa ttc cat tac aac acc gtt gtt gat aat atc ttt gtt      768
   Arg Gly Val Gln Phe His Tyr Asn Thr Val Val Asp Asn Ile Phe Val
                   245                 250                 255
   aac cgt tca aat ggt gaa aag att gct aag caa att ctt tta act gaa      816
   Asn Arg Ser Asn Gly Glu Lys Ile Ala Lys Gln Ile Leu Leu Thr Glu
               260                 265                 270
   aac ggt gaa aaa aag agc atc gat tta aca gaa aat gac ctc gtc ttc      864
   Asn Gly Glu Lys Lys Ser Ile Asp Leu Thr Glu Asn Asp Leu Val Phe
           275                 280                 285
   gtt act aac ggt tca att act gaa agt aca act tat ggt gat aac ttg      912
   Val Thr Asn Gly Ser Ile Thr Glu Ser Thr Thr Tyr Gly Asp Asn Leu
       290                 295                 300
   cac cca gct tct gag gaa cat aaa tta ggt gct act tgg aaa tta tgg      960
   His Pro Ala Ser Glu Glu His Lys Leu Gly Ala Thr Trp Lys Leu Trp
   305                 310                 315                 320
   caa aac ttg gca gcg caa gat gat gac ttc ggt cac cca gat gtc ttc     1008
   Gln Asn Leu Ala Ala Gln Asp Asp Asp Phe Gly His Pro Asp Val Phe
                   325                 330                 335
   tgc aag gat att cca aag gct aac tgg gta atg tct gct aca att act     1056
   Cys Lys Asp Ile Pro Lys Ala Asn Trp Val Met Ser Ala Thr Ile Thr
               340                 345                 350
   ttt aag aat aat gat att gtg cca ttc att gaa gca gtt aat aag aag     1104
   Phe Lys Asn Asn Asp Ile Val Pro Phe Ile Glu Ala Val Asn Lys Lys
           355                 360                 365
   gat cca cac agc ggc tca att gta act agt ggg cct act acg att aag     1152
   Asp Pro His Ser Gly Ser Ile Val Thr Ser Gly Pro Thr Thr Ile Lys
       370                 375                 380
   gat tct aac tgg cta ctt ggt tat tca atc agt cgt cag cct cac ttt      1200
   Asp Ser Asn Trp Leu Leu Gly Tyr Ser Ile Ser Arg Gln Pro His Phe
   385                 390                 395                 400
   gaa gca caa aag cct aac gaa ttg att gta tgg ctt tat ggt ttg ttc      1248
   Glu Ala Gln Lys Pro Asn Glu Leu Ile Val Trp Leu Tyr Gly Leu Phe
                   405                 410                 415
   tca gac acc aaa ggt aac tat gtt gaa aag act atg cct gac tgt aac      1296
   Ser Asp Thr Lys Gly Asn Tyr Val Glu Lys Thr Met Pro Asp Cys Asn
               420                 425                 430
   ggt att gaa tta tgt gaa gaa tgg ctt tac cac atg ggt gtt cct gaa      1344
   Gly Ile Glu Leu Cys Glu Glu Trp Leu Tyr His Met Gly Val Pro Glu
           435                 440                 445
   gaa aga atc cca gaa atg gct tca gct gct acg act att cca gca cac      1392
   Glu Arg Ile Pro Glu Met Ala Ser Ala Ala Thr Thr Ile Pro Ala His
       450                 455                 460
   atg cca tat att act tca tac ttc atg cca aga gca tta ggc gac aga      1440
   Met Pro Tyr Ile Thr Ser Tyr Phe Met Pro Arg Ala Leu Gly Asp Arg
   465                 470                 475                 480
   ccc aag gtt gtg cca gac cac tca aag aac ttg gcc ttc att ggt aac      1488
   Pro Lys Val Val Pro Asp His Ser Lys Asn Leu Ala Phe Ile Gly Asn
                   485                 490                 495
   ttt gct gaa acg cca aga gac act gtc ttt acc act gaa tac tct gtc      1536
   Phe Ala Glu Thr Pro Arg Asp Thr Val Phe Thr Thr Glu Tyr Ser Val
               500                 505                 510
   aga act gcg atg gaa gct gta tac acc ttg ctt aac att gat cgt ggt      1584
   Arg Thr Ala Met Glu Ala Val Tyr Thr Leu Leu Asn Ile Asp Arg Gly
           515                 520                 525
   gtg cca gaa gta ttt gca tct gcc ttc gat gtc aga atg ctc atg aac      1632
   Val Pro Glu Val Phe Ala Ser Ala Phe Asp Val Arg Met Leu Met Asn
       530                 535                 540
   gca atg tac tac ttg aat gat caa aag aag ctt gaa gat ctt gat ttg      1680
   Ala Met Tyr Tyr Leu Asn Asp Gln Lys Lys Leu Glu Asp Leu Asp Leu
   545                 550                 555                 560
   cct att gct gaa aag ttg gca att aag ggg atg ctc aag aaa gtt aag      1728
   Pro Ile Ala Glu Lys Leu Ala Ile Lys Gly Met Leu Lys Lys Val Lys
                   565                 570                 575
   ggc act tat ata gag gaa ttg ctt aag aag tat aag ttg gtt tag          1773
   Gly Thr Tyr Ile Glu Glu Leu Leu Lys Lys Tyr Lys Leu Val
               580                 585                 590
<210> 2
   <211> 590
   <212> PRT
   <213> Lactobacillus acidophilus
<400> 2
   Met His Tyr Ser Ser Gly Asn Tyr Glu Ala Phe Val Asn Ala Ser Lys
   1               5                   10                  15
   Pro Lys Asp Val Asp Gln Lys Ser Ala Tyr Leu Val Gly Ser Gly Leu
               20                  25                  30
   Ala Ser Leu Ala Ser Ala Val Phe Leu Ile Arg Asp Gly His Met Lys
           35                  40                  45
   Gly Asp Arg Ile His Ile Leu Glu Glu Leu Ser Leu Pro Gly Gly Ser
       50                  55                  60
   Met Asp Gly Ile Tyr Asn Lys Gln Lys Glu Ser Tyr Ile Ile Arg Gly
   65                  70                  75                  80
   Gly Arg Glu Met Glu Ala His Phe Glu Cys Leu Trp Asp Leu Phe Arg
                   85                  90                  95
   Ser Ile Pro Ser Ala Glu Asn Lys Asp Glu Ser Val Leu Asp Glu Phe
               100                 105                 110
   Tyr Arg Leu Asn Arg Lys Asp Pro Ser Phe Ala Lys Thr Arg Val Ile
           115                 120                 125
   Val Asn Arg Gly His Glu Leu Pro Thr Asp Gly Gln Leu Leu Leu Thr
       130                 135                 140
   Pro Lys Ala Val Lys Glu Ile Ile Asp Leu Cys Leu Thr Pro Glu Lys
   145                 150                 155                 160
   Asp Leu Gln Asn Lys Lys Ile Asn Glu Val Phe Ser Lys Glu Phe Phe
                   165                 170                 175
   Glu Ser Asn Phe Trp Leu Tyr Trp Ser Thr Met Phe Ala Phe Glu Pro
               180                 185                 190
   Trp Ala Ser Ala Met Glu Met Arg Arg Tyr Leu Met Arg Phe Val Gln
           195                 200                 205
   His Val Ser Thr Leu Lys Asn Leu Ser Ser Leu Arg Phe Thr Lys Tyr
       210                 215                 220
   Asn Gln Tyr Glu Ser Leu Ile Leu Pro Met Val Lys Tyr Leu Lys Asp
   225                 230                 235                 240
   Arg Gly Val Gln Phe His Tyr Asn Thr Val Val Asp Asn Ile Phe Val
                   245                 250                 255
   Asn Arg Ser Asn Gly Glu Lys Ile Ala Lys Gln Ile Leu Leu Thr Glu
               260                 265                 270
   Asn Gly Glu Lys Lys Ser Ile Asp Leu Thr Glu Asn Asp Leu Val Phe
           275                 280                 285
   Val Thr Asn Gly Ser Ile Thr Glu Ser Thr Thr Tyr Gly Asp Asn Leu
       290                 295                 300
   His Pro Ala Ser Glu Glu His Lys Leu Gly Ala Thr Trp Lys Leu Trp
   305                 310                 315                 320
   Gln Asn Leu Ala Ala Gln Asp Asp Asp Phe Gly His Pro Asp Val Phe
                   325                 330                 335
   Cys Lys Asp Ile Pro Lys Ala Asn Trp Val Met Ser Ala Thr Ile Thr
               340                 345                 350
   Phe Lys Asn Asn Asp Ile Val Pro Phe Ile Glu Ala Val Asn Lys Lys
           355                 360                 365
   Asp Pro His Ser Gly Ser Ile Val Thr Ser Gly Pro Thr Thr Ile Lys
       370                 375                 380
   Asp Ser Asn Trp Leu Leu Gly Tyr Ser Ile Ser Arg Gln Pro His Phe
   385                 390                 395                 400
   Glu Ala Gln Lys Pro Asn Glu Leu Ile Val Trp Leu Tyr Gly Leu Phe
                   405                 410                 415
   Ser Asp Thr Lys Gly Asn Tyr Val Glu Lys Thr Met Pro Asp Cys Asn
               420                 425                 430
   Gly Ile Glu Leu Cys Glu Glu Trp Leu Tyr His Met Gly Val Pro Glu
           435                 440                 445
   Glu Arg Ile Pro Glu Met Ala Ser Ala Ala Thr Thr Ile Pro Ala His
       450                 455                 460
   Met Pro Tyr Ile Thr Ser Tyr Phe Met Pro Arg Ala Leu Gly Asp Arg
   465                 470                 475                 480
   Pro Lys Val Val Pro Asp His Ser Lys Asn Leu Ala Phe Ile Gly Asn
                   485                 490                 495
   Phe Ala Glu Thr Pro Arg Asp Thr Val Phe Thr Thr Glu Tyr Ser Val
               500                 505                 510
   Arg Thr Ala Met Glu Ala Val Tyr Thr Leu Leu Asn Ile Asp Arg Gly
               515                 520                 525
   Val Pro Glu Val Phe Ala Ser Ala Phe Asp Val Arg Met Leu Met Asn
       530                 535                 540
   Ala Met Tyr Tyr Leu Asn Asp Gln Lys Lys Leu Glu Asp Leu Asp Leu
   545                 550                 555                 560
   Pro Ile Ala Glu Lys Leu Ala Ile Lys Gly Met Leu Lys Lys Val Lys
                   565                 570                 575
   Gly Thr Tyr Ile Glu Glu Leu Leu Lys Lys Tyr Lys Leu Val
               580                 585                 590

## Description

### Technical Field

The present invention relates to an agent containing a rare fatty acid as defined herein for use for the prophylaxis or treatment of an inflammatory disease, more particularly wherein said agent utilizes the physiological function, for example, an action to suppress an inflammation reaction in vivo, of rare fatty acids such as oxo fatty acid, hydroxy fatty acid and the like as defined herein. The present invention also relates to said agent for use, wherein said agent is a food, a pharmaceutical product, a feed and the like containing the agent.

### Background Art

Inflammation is a defense reaction of living organisms which is observed when biological tissues are injured by bacterial infection, action of physicochemical factor and the like, wherein the causal substance of injury and injured tissues are removed thereby. As regards inflammatory reaction in living organisms, it is known that nitric oxide (NO) produced by M1 macrophage acts as a mediator. While NO acts as a biological defense factor, it exacerbates inflammation when produced in large amounts during inflammation. For example, in adipose tissue in obese state, inflammation is promoted by mutual activation of activated M1 macrophage and adipocyte and a disease state is induced. Therefore, an anti-inflammatory action of a substance that suppresses NO production by M1 macrophage is expected.

As a fatty acid derivative showing an anti-inflammatory action, hydroxylated fatty acids produced by lipoxygenase have been reported. For example, when a plant-derived lipoxygenase is used, 9-hydroxy-trans-10,cis-12-octadecadienoic acid, 13-hydroxy-cis-9,trans-11-octadecadienoic acid are produced from linoleic acid; 13-hydroxy-10-oxo-trans-11-octadecenoic acid, 9-hydroxy-13-oxo-trans-10-octadecenoic acid, 9-hydroxy-10-oxo-cis-12-octadecenoic acid, 13-hydroxy-12-oxo-cis-9-octadecenoic acid are further produced by a combined use with hydroxy peroxide isomerase, and these hydroxylated fatty acids and oxo fatty acids are reported to have an anti-inflammatory action (patent document 1). Also, as endogenous anti-inflammatory lipid mediator in mammals, epoxy form and hydroxylated form of ω3 fatty acids such as eicosapentaenoic acid (EPA), docosahexaenoic acid (DHA) and the like, which are produced by the action of oxidizing enzymes such as lipoxygenase, monooxygenase and the like, have been reported (patent document 2) .

As for rare fatty acids such as hydroxylated fatty acid, oxo fatty acid and the like, since a part of oxo fatty acids such as 9-oxo-octadecadienoic acid, 13-oxo-octadecadienoic acid and the like contained in tomato have been reported to have an activity to improve lifestyle-related diseases, such as lipid metabolism improvement and the like (patent document 3, non-patent documents 1, 2), the physiological functions of rare fatty acids are drawing attention.

As regards production of rare fatty acids such as oxo fatty acid, hydroxylated fatty acid and the like, a production method of C10-position hydroxylated fatty acid and C10-position oxo fatty acid, each having 18 carbon atoms, which uses hydration dehydrase derived from lactobacillus plantarum, and which was found by the inventors, has been reported (patent document 4). In addition, a metabolism improving effect (patent document 5), and an intestine protective action (patent document 6) relating to these C10-position hydroxylated fatty acid and C10-position oxo fatty acid have also been reported. However, the physiological functions other than metabolism improvement and intestinal protection, for example, physiological functions such as anti-inflammatory action and the like, of these hydroxylated fatty acid, oxo fatty acid, as well as hydroxylated fatty acid and oxo fatty acid, each having a carbon atom number other than 18 or hydroxylated fatty acid and oxo fatty acid, each having hydroxyl group, carbonyl group at a position other than C10-position are unknown.

### [Document List]

### [Patent Documents]

patent document 1: JP-A-2005-008594
patent document 2: WO 2012/023254
patent document 3: JP-A-2011-184411
patent document 4: WO 2013/168310
patent document 5: WO 2014/069227
patent document 6: WO 2014/129384

### [non-patent document]

non-patent document 1: Kim Y-I, (2011), Mol. Nutr. Food Res., vol.55, p.585-593
non-patent document 2: Kim Y-I,(2012), PLoS ONE, vol.7, no.2, e31317

### SUMMARY OF THE INVENTION

### Problems to be Solved by the Invention

An object of the present invention is to provide an anti-inflammatory agent containing a rare fatty acid as defined herein, which suppresses an inflammatory reaction, for use for the prophylaxis or treatment of an inflammatory disease.

### Means of Solving the Problems

The present inventors have conducted intensive studies in view of the above-mentioned problems and found that rare fatty acids such as a C10-position hydroxylated fatty acid having 18 carbon atoms and a C10-position oxo fatty acid having 18 carbon atoms as defined herein, obtained using a fatty acid saturating enzyme group derived from Lactobacillus plantarum and a chemical oxidation reaction, and a C13-position hydroxylated fatty acid having 18 carbon atoms and a C13-position oxo fatty acid having 18 carbon atoms as defined herein, obtained using a hydration enzyme derived from Lactobacillus acidophilus and a chemical oxidation reaction and the like, which were found by the inventors, have a conventionally-unknown anti-inflammatory action based on an action to suppress NO production by M1 macrophage. In addition, the present inventors have found that the above-mentioned rare fatty acids have an effect to increase resistance of cells to H₂O₂ stress, and an effect to increase expression of an anti-oxidant enzyme HO-1 mRNA. Furthermore, the present inventors have found that the above-mentioned rare fatty acids suppress differentiation of monocyte and macrophage into M1 macrophage by directly or indirectly inducing differentiation of monocyte and macrophage into M2 macrophage.

The present invention has been completed based on the above findings.

Accordingly, the present invention provides the following:
[1] An agent comprising the following fatty acid:
   (1) a saturated fatty acid or an unsaturated fatty acid having a trans double bond at the 11-position or at least one cis double bond at the 6-position, the 12-position, the 15-position, which has 18 carbon atoms and a hydroxyl group or carbonyl group at the 10-position selected from the group consisting of 10-hydroxy-cis-12-octadecenoic acid, 10-hydroxy-cis-12,cis-15-octadecadienoic acid, 10-hydroxy-cis-6,cis-12-octadecadienoic acid, 10-hydroxy-cis-6,cis-12,cis-15-octadecatrienoic acid, 10,12-dihydroxy-octadecanoic acid, 10-hydroxy-octadecanoic acid, 10-hydroxy-cis-15-octadecenoic acid, 10-hydroxy-cis-6-octadecenoic acid, 10-hydroxy-cis-6,cis-15-octadecadienoic acid, 10-hydroxy-trans-11-octadecenoic acid, 10-hydroxy-trans-11,cis-15-octadecadienoic acid, 10-hydroxy-cis-6,trans-11-octadecadienoic acid, 10-hydroxy-cis-6,trans-11,cis-15-octadecatrienoic acid, 10-oxo-cis-12-octadecenoic acid, 10-oxo-cis-12,cis-15-octadecadienoic acid, 10-oxo-cis-6,cis-12-octadecadienoic acid, 10-oxo-cis-6,cis-12,cis-15-octadecatrienoic acid, 10-oxo-octadecanoic acid, 10-oxo-cis-6-octadecenoic acid, 10-oxo-cis-15-octadecenoic acid, 10-oxo-cis-6,cis-15-octadecadienoic acid, 10-oxo-cis-6,trans-11-octadecadienoic acid, 10-oxo-trans-11,cis-15-octadecadienoic acid or 10-oxo-cis-6,trans-11,cis-15-octadecatrienoic acid,
   (2) a saturated fatty acid or an unsaturated fatty acid having at least one cis double bond at the 6-position, the 9-position, the 15-position, which has 18 carbon atoms and a hydroxyl group or carbonyl group at the 13-position selected from the group consisting of 13-hydroxy-cis-9-octadecenoic acid, 13-hydroxy-cis-6,cis-9-octadecadienoic acid, 13-hydroxy-cis-9,cis-15-octadecadienoic acid, 13-hydroxy-cis-6,cis-9,cis-15-octadecatrienoic acid, 10,13-dihydroxy-octadecanoic acid, 10,13-dihydroxy-cis-6-octadecenoic acid, 10,13-dihydroxy-cis-15-octadecenoic acid, 10,13-dihydroxy-cis-6,cis-15-octadecadienoic acid, 10-oxo-13-hydroxy-cis-6-octadecenoic acid, 10-oxo-13-hydroxy-cis-15-octadecenoic acid, 10-oxo-13-hydroxy-cis-6,cis-15-octadecadienoic acid, 13-hydroxy-cis-5,cis-9-octadecadienoic acid, 13-hydroxy-trans-5,cis-9-octadecadienoic acid, 13-oxo-cis-9-octadecenoic acid, 13-oxo-cis-6,cis-9-octadecadienoic acid, 13-oxo-cis-9,cis-15-octadecadienoic acid, 13-oxo-cis-6,cis-9,cis-15-octadecatrienoic acid, 10,13-dioxo-octadecanoic acid, 10,13-dioxo-cis-6-octadecenoic acid, 10,13-dioxo-cis-15-octadecenoic acid, 10,13-dioxo-cis-6,cis-15-octadecadienoic acid, 13-oxo-cis-5,cis-9-octadecadienoic acid or 13-oxo-trans-5,cis-9-octadecadienoic acid,
   (3) a saturated fatty acid or an unsaturated fatty acid having at least one cis double bond at the 5-position, the 8-position, the 9-position, the 14-position, the 17-position, which has 18 or 20 carbon atoms and a hydroxyl group or carbonyl group at the 12-position selected from the group consisting of 12-hydroxy-cis-14-eicosenoic acid, 12-hydroxy-cis-14,cis-17-eicosadienoic acid, 12-hydroxy-cis-8,cis-14-eicosadienoic acid, 12-hydroxy-cis-5,cis-8-eicosadienoic acid, 12-hydroxy-cis-8,cis-14,cis-17-eicosatrienoic acid, 12-hydroxy-cis-5,cis-8,cis-14-eicosatrienoic acid, 12-oxo-octadecanoic acid, 12-oxo-cis-14-eicosenoic acid, 12-oxo-cis-14,cis-17-eicosadienoic acid, 12-oxo-cis-8,cis-14-eicosadienoic acid, 12-oxo-cis-5,cis-8-eicosadienoic acid, 12-oxo-cis-8,cis-14,cis-17-eicosatrienoic acid, or 12-oxo-cis-5,cis-8,cis-14-eicosatrienoic acid,
   (4) an unsaturated fatty acid having at least one cis double bond at the 5-position, the 8-position, the 11-position, the 17-position, which has 20 carbon atoms and a hydroxyl group or carbonyl group at the 15-position selected from the group consisting of 15-hydroxy-cis-11-eicosenoic acid, 15-hydroxy-cis-11,cis-17-eicosadienoic acid, 15-hydroxy-cis-8,cis-11-eicosadienoic acid, 15-hydroxy-cis-5,cis-8,cis-11-eicosatrienoic acid, 15-hydroxy-cis-8,cis-11,cis-17-eicosatrienoic acid, 15-hydroxy-cis-5,cis-11-eicosadienoic acid, 15-hydroxy-cis-5,cis-11,cis-17-eicosatrienoic acid, 15-oxo-cis-11-eicosenoic acid, 15-oxo-cis-11,cis-17-eicosadienoic acid, 15-oxo-cis-8,cis-11-eicosadienoic acid, 15-oxo-cis-5,cis-8,cis-11-eicosatrienoic acid, 15-oxo-cis-8,cis-11,cis-17-eicosatrienoic acid, 15-oxo-cis-5,cis-11-eicosadienoic acid or 15-oxo-cis-5,cis-11,cis-17-eicosatrienoic acid, or
   (5) a saturated fatty acid having 16 carbon atoms and a hydroxyl group or carbonyl group at the 10-position selected from the group consisting of 10-hydroxy-hexadecanoic acid or 10-oxo-hexadecanoic acid,
      for use for the prophylaxis or treatment of an inflammatory disease;
[2] the agent for use according to [1], comprising the following fatty acid:
   (1) a saturated fatty acid or an unsaturated fatty acid having a trans double bond at the 11-position or at least one cis double bond at the 6-position, the 12-position, which has 18 carbon atoms and a hydroxyl group or carbonyl group at the 10-position selected from the group consisting of 10-hydroxy-cis-12-octadecenoic acid, 10-hydroxy-octadecanoic acid, 10-oxo-cis-12-octadecenoic acid, 10-hydroxy-cis-12,cis-15-octadecadienoic acid, 10-hydroxy-cis-6,cis-12-octadecadienoic acid, 10-oxo-cis-12,cis-15-octadecadienoic acid, 10-oxo-cis-6,cis-12-octadecadienoic acid, 10-oxo-trans-11,cis-15-octadecadienoic acid or 10-oxo-cis-6,trans-11-octadecadienoic acid, or
   (2) a saturated fatty acid or an unsaturated fatty acid having at least one cis double bond at the 6-position, the 9-position, the 15-position, which has 18 carbon atoms and a hydroxyl group or carbonyl group at the 13-position selected from the group consisting of 13-hydroxy-cis-9-octadecenoic acid, 13-hydroxy-cis-6,cis-9-octadecadienoic acid, 13-hydroxy-cis-9,cis-15-octadecadienoic acid, 10,13-dihydroxy-octadecanoic acid, 10,13-dihydroxy-cis-6-octadecenoic acid, 10,13-dihydroxy-cis-15-octadecenoic acid, 13-oxo-cis-9-octadecenoic acid, 13-oxo-cis-6,cis-9-octadecadienoic acid, or 13-oxo-cis-9,cis-15-octadecadienoic acid;
[3] the agent for use according to [1] or [2] for use for the prophylaxis or improvement of an inflammatory disease involving macrophage;
[4] the agent for use according to [1] or [2], wherein said agent is a food or a food additive;
[8] the agent for use according to [1] or [2], wherein said agent is a pharmaceutical product;
[9] the agent for use according to [1] or [2], wherein said agent is a feed or a feed additive.

### [Effect of the Invention]

In the present invention, it was found that hydroxylated fatty acids such as 10-hydroxy-cis-12-octadecenoic acid and 13-hydroxy-cis-9-octadecenoic acid or oxo fatty acids such as 10-oxo-cis-12-octadecenoic acid and 13-oxo-cis-9-octadecenoic acid (hereinafter to be also referred to as rare fatty acid derivative) have an anti-inflammatory action which is a physiological function conventionally not known.

The present invention provides an anti-inflammatory agent containing a rare fatty acid derivative such as hydroxylated fatty acid and the like as defined herein for use for the prophylaxis or treatment of an inflammatory disease, based on the function.

### [Brief Description of the Drawings]

Fig. 1 shows the measurement results of NO production amount by the Griess method, wherein Cont. shows negative control (ethanol addition), Posi. shows positive control (IκBα phosphorylation inhibitor addition), and the vertical axis shows NO production amount.
Fig. 2 shows the measurement results of cell survival rate relative to H₂O₂. A: Various compounds (No. 11 - No. 13) were each used at 30 µM. B: Compound No. 11 was used at 10, 20, 30 µM. Nor. shows cells free of an H₂O₂ treatment, Con. shows negative control, tBHQ shows positive control, and the vertical axis shows cell survival rate.
Fig. 3 shows the measurement results of anti-oxidant enzyme HO-1 mRNA expression. A: Various compounds (No. 11 - No. 13) were each used at 30 µM. B: Compound No. 11 was used at 10, 30, 60, 90 µM. Con. shows negative control, tBHQ shows positive control, and the vertical axis shows relative expression level of HO-1 mRNA.
Fig. 4 shows Western blot images and a graph showing the measurement results of intranuclear expression of transcription factor Nrf2. Con. shows negative control, tBHQ shows positive control, and the vertical axis shows relative expression level of intranuclear Nrf2.
Fig. 5 shows the measurement results of transcription activity of transcription factor Nrf2. Con. shows negative control, tBHQ shows positive control, and the vertical axis shows relative luciferase activity.
Fig. 6 shows a culture schedule for inducing differentiation of mouse bone marrow-derived cells into M2 macrophage.
Fig. 7 shows the proportion of cells having cellular surface antigen of M2 macrophage.
Fig. 8 shows the proportion of cells having cellular surface antigen of M2 macrophage.
Fig. 9 shows the proportion of cells having cellular surface antigen of M2 macrophage.
Fig. 10 shows the proportion of cells having cellular surface antigen of M2 macrophage.
Fig. 11 shows an Arginase 1 mRNA expression level.
Fig. 12 shows an IL-1β mRNA expression level.
Fig. 13 shows a culture schedule for inducing differentiation of mouse bone marrow-derived cells into M2 macrophage.
Fig. 14 shows the proportion of cells having cellular surface antigen of M2 macrophage.
Fig. 15 shows the proportion of cells having cellular surface antigen of M2 macrophage.
Fig. 16 shows the expression level of Arginase 1 mRNA.
Fig. 17 shows the expression level of IL-1β mRNA.
Fig. 18 shows the IL-4 expression level.
Fig. 19 shows the MCP-1 expression level.

### [Description of Embodiments]

The present invention is explained in detail below.

In the present invention, "anti-inflammatory" means suppression of inflammation in vivo. To be specific, an "anti-inflammatory agent" means, for example, prophylaxis and/or suppression of inflammation such as tissue injury (rheumatism, arteriosclerosis etc.) due to allergic disease, chronic inflammation and nerve inflammation disease.

As an index of the above-mentioned anti-inflammatory activity, nitric oxide (NO) production by M1 macrophage can be measured. While macrophage is one kind of immunocyte that plays a central role in the biological defense mechanism against biological exposure to foreign substances such as pathogenic bacteria and the like that invade from the outer world, when macrophage is activated, it becomes M1 macrophage, and produces inflammatory mediators including NO, prostaglandin E2, cytokines such as TNF-α and the like. Promoted production of these inflammatory mediators causes tissue disorders due to chronic inflammation, and induces arteriosclerosis, rheumatism and the like. Therefore, whether NO production by M1 macrophage activated by lipopolysaccharide (LPS) is suppressed by the below-mentioned rare fatty acid derivative of the present invention is confirmed and, when NO production is suppressed, the rare fatty acid derivative can be judged to highly possibly have an anti-inflammatory effect, or have an anti-inflammatory effect. As one example, the NO production amount can be evaluated by the Griess method, though the method is not limited.

As an index of the above-mentioned anti-inflammatory activity, moreover, the cell survival rate relative to H₂O₂ can be measured. H₂O₂ as an active oxygen promotes production of inflammatory cytokine, and causes inflammatory diseases like the above-mentioned M1 macrophage. Reduction of oxidative stress by H₂O₂ affords an anti-inflammatory action. Therefore, whether the survival rate of cultured cells relative to H₂O₂ increases by the below-mentioned rare fatty acid derivative of the present invention is confirmed and, when the survival rate increased, the rare fatty acid derivative can be judged to highly possibly have an anti-inflammatory effect, or have an anti-inflammatory effect.

As an index of the above-mentioned anti-inflammatory activity, moreover, promotion of the expression of heme oxygenase (HO-1) can also be measured. HO-1 is an important enzyme as a defense mechanism in the body that protects cells from oxidative stress. Lack of HO-1 promotes cell injury due to oxidative stress, promotes inflammation; on the other hand, promoted expression of HO-1 suppresses oxidative stress, and suppresses inflammation due to cell injury. Therefore, whether the expression of HO-1 in the cultured cells is promoted by the below-mentioned rare fatty acid derivative of the present invention is confirmed and, when the HO-1 expression was promoted, the rare fatty acid derivative can be judged to highly possibly have an anti-inflammatory effect, or have an anti-inflammatory effect.

Alternatively, as an index of the above-mentioned anti-inflammatory activity, promotion of the expression of intranuclear transcription factor Nrf2 can also be measured. The intranuclear expression of Nrf2 is promoted and activated by electrophilic substance, active oxygen, endoplasmic reticulum stress and the like, and Nrf2 controls oxidative stress-adaptive reaction and suppresses inflammation in higher animals. Therefore, whether the intranuclear expression of Nrf2 in the cultured cells is promoted by the below-mentioned rare fatty acid derivative of the present invention is confirmed and, when the intranuclear expression of Nrf2 was promoted, the rare fatty acid derivative can be judged to highly possibly have an anti-inflammatory effect, or have an anti-inflammatory effect. Alternatively, whether the transcription activity of Nrf2 in the cultured cells is promoted by the below-mentioned rare fatty acid derivative of the present invention is confirmed and, when the transcription activity of Nrf2 was promoted, the rare fatty acid derivative can be judged to highly possibly have an anti-inflammatory effect, or have an anti-inflammatory effect.

Alternatively, as an index of the above-mentioned anti-inflammatory activity, an expression marker of M2 macrophage can also be measured. M1 macrophage is activated on infection with bacterium, virus or fungi, and produces a tumor necrosis factor (TNF), nitric oxide, and cytokines such as IL-6, IFN, IL-1β and the like, which are important for the elimination of such pathogens. On the other hand, M2 macrophage is involved in parasitic infection, allergic response, fat metabolism, wound therapy, cancer metastasis and the like. It is known that macrophage is differentiated into M1 or M2, and the proportion of M1 can be decreased by inducing differentiation into M2, as a result of which inflammation is suppressed. Therefore, whether expression of cellular surface antigen expressed in M2 macrophage and expression of mRNA marker expressed in M2 macrophage are promoted by the below-mentioned rare fatty acid derivative of the present invention is confirmed and, when they were promoted, the rare fatty acid derivative can be judged to highly possibly have an anti-inflammatory effect, or have an anti-inflammatory effect.

In the present invention, the rare fatty acid derivative refers to a rare fatty acid derivative that can be produced using a fatty acid saturating enzyme group derived from Lactobacillus plantarum and a chemical oxidation reaction (hereinafter sometimes to be abbreviated as "LP-rare fatty acid derivative"), and a rare fatty acid derivative that can be produced using a hydration enzyme derived from Lactobacillus acidophilus and a chemical oxidation reaction (hereinafter sometimes to be abbreviated as "LA-rare fatty acid derivative").

The LP-rare fatty acid derivative of the present invention refers to a fatty acid having 18 carbon atoms and a hydroxyl group or carbonyl group at the 10-position. As used herein, the fatty acid having 18 carbon atoms and a hydroxyl group or carbonyl group at the 10-position refers to a hydroxylated fatty acid having 18 carbon atoms and a hydroxyl group at the 10-position (hereinafter sometimes to be abbreviated as "10-hydroxy fatty acid"), or oxo fatty acid having 18 carbon atoms and a carbonyl group at the 10-position (hereinafter sometimes to be abbreviated as "10-oxo fatty acid"). The fatty acid having 18 carbon atoms and a hydroxyl group or carbonyl group at the 10-position is a saturated fatty acid or an unsaturated fatty acid having a trans double bond at the 11-position, or at least one cis double bond at the 6-position, the 12-position, the 15-position. The unsaturated fatty acid may further has a cis double bond at the 6-position or 15-position.

More specifically, the saturated fatty acid or the unsaturated fatty acid having a trans double bond at the 11-position or at least one cis double bond at the 6-position, the 12-position, the 15-position, which has 18 carbon atoms and a hydroxyl group or carbonyl group at the 10-position (preferably, a saturated fatty acid or an unsaturated fatty acid having a trans double bond at the 11-position or at least one cis double bond at the 6-position, the 12-position, which has 18 carbon atoms and a hydroxyl group or carbonyl group at the 10-position) is selected from the group consisting of 10-hydroxy-cis-12-octadecenoic acid, 10-hydroxy-cis-12,cis-15-octadecadienoic acid, 10-hydroxy-cis-6,cis-12-octadecadienoic acid, 10-hydroxy-cis-6,cis-12,cis-15-octadecatrienoic acid, 10,12-dihydroxy-octadecanoic acid, 10-hydroxy-octadecanoic acid, 10-hydroxy-cis-15-octadecenoic acid, 10-hydroxy-cis-6-octadecenoic acid, 10-hydroxy-cis-6,cis-15-octadecadienoic acid, 10-hydroxy-trans-11-octadecenoic acid, 10-hydroxy-trans-11,cis-15-octadecadienoic acid, 10-hydroxy-cis-6,trans-11-octadecadienoic acid, 10-hydroxy-cis-6,trans-11,cis-15-octadecatrienoic acid, 10-oxo-cis-12-octadecenoic acid, 10-oxo-cis-12,cis-15-octadecadienoic acid, 10-oxo-cis-6,cis-12-octadecadienoic acid, 10-oxo-cis-6,cis-12,cis-15-octadecatrienoic acid, 10-oxooctadecanoic acid, 10-oxo-cis-6-octadecenoic acid, 10-oxo-cis-15-octadecenoic acid, 10-oxo-cis-6,cis-15-octadecadienoic acid, 10-oxo-cis-6,trans-11-octadecadienoic acid, 10-oxo-trans-11,cis-15-octadecadienoic acid and 10-oxo-cis-6,trans-11,cis-15-octadecatrienoic acid, preferably, 10-hydroxy-cis-12-octadecenoic acid, 10-hydroxy-octadecanoic acid, 10-oxo-cis-12-octadecenoic acid, 10-hydroxy-cis-12,cis-15-octadecadienoic acid, 10-hydroxy-cis-6,cis-12-octadecadienoic acid, 10-oxo-cis-12,cis-15-octadecadienoic acid, 10-oxo-cis-6,cis-12-octadecadienoic acid, 10-oxo-trans-11,cis-15-octadecadienoic acid or 10-oxo-cis-6,trans-11-octadecadienoic acid, more preferably, 10-oxo-trans-11,cis-15-octadecadienoic acid, or 10-oxo-cis-6,trans-11-octadecadienoic acid.

The LA-rare fatty acid derivative in the present invention refers to a fatty acid having 18 carbon atoms and a hydroxyl group or carbonyl group at the 13-position, a fatty acid having 16 carbon atoms and a hydroxyl group or carbonyl group at the 10-position, a fatty acid having 20 carbon atoms and a hydroxyl group or carbonyl group at the 15-position or a fatty acid having 18 or 20 carbon atoms and a hydroxyl group or carbonyl group at the 12-position.

In the present invention, a fatty acid having 18 carbon atoms and a hydroxyl group or carbonyl group at the 13-position refers to a hydroxylated fatty acid having 18 carbon atoms and a hydroxyl group at the 13-position (hereinafter sometimes to be abbreviated as "13-hydroxy fatty acid"), or an oxo fatty acid having 18 carbon atoms and a carbonyl group at the 13-position (hereinafter sometimes to be abbreviated as "13-oxo fatty acid"). As used herein, a fatty acid having 18 carbon atoms and a hydroxyl group or carbonyl group at the 10-position and the 13-position (hereinafter sometimes to be abbreviated as "10,13-dihydroxy fatty acid" or "10,13-dioxo fatty acid"), and a fatty acid having 18 carbon atoms and a hydroxyl group at the 13-position and a carbonyl group at the 10-position (hereinafter sometimes to be abbreviated as "10-oxo-13-hydroxy fatty acid") are also encompassed in one embodiment of the "13-hydroxy fatty acid" or "13-oxo fatty acid". In addition, the fatty acid having 18 carbon atoms and a hydroxyl group or carbonyl group at the 13-position is a saturated fatty acid or an unsaturated fatty acid having at least one cis double bond at the 6-position, 9-position, the 15-position. When the fatty acid having 18 carbon atoms and a hydroxyl group or carbonyl group at the 13-position is an unsaturated fatty acid, an unsaturated fatty acid having a cis double bond at the 9-position is preferable.

In the present invention, moreover, the fatty acid having 16 carbon atoms and a hydroxyl group or a carbonyl group at the 10-position refers to a hydroxylated fatty acid having 16 carbon atoms and a hydroxyl group at the 10-position (hereinafter sometimes to be abbreviated as "10-hydroxy fatty acid"), or oxo fatty acid having 16 carbon atoms and a carbonyl group at the 10-position (hereinafter sometimes to be abbreviated as "10-oxo fatty acid"). In addition, the fatty acid having 16 carbon atoms and a hydroxyl group or a carbonyl group at the 10-position is a saturated fatty acid or an unsaturated fatty acid.

In the present invention, the fatty acid having 20 carbon atoms and a hydroxyl group or carbonyl group at the 15-position refers to a hydroxylated fatty acid having 20 carbon atoms and a hydroxyl group at the 15-position (hereinafter sometimes to be abbreviated as "15-hydroxy fatty acid"), or oxo fatty acid having 20 carbon atoms and a carbonyl group at the 15-position fatty acid (hereinafter sometimes to be abbreviated as "15-oxo fatty acid"). In addition, the fatty acid having 20 carbon atoms and a hydroxyl group or carbonyl group at the 15-position is a saturated fatty acid or an unsaturated fatty acid having at least one cis double bond at the 5-position, the 8-position, the 11-position, the 17-position.

In the present invention, the fatty acid having 18 or 20 carbon atoms and a hydroxyl group or carbonyl group at the 12-position refers to a hydroxylated fatty acid having 18 or 20 carbon atoms and a hydroxyl group at the 12-position (hereinafter sometimes to be abbreviated as "12-hydroxy fatty acid"), or oxo fatty acid having 18 or 20 carbon atoms and a carbonyl group at the 12-position (hereinafter sometimes to be abbreviated as "12-oxo fatty acid"). In addition, the fatty acid having 18 or 20 carbon atoms and a hydroxyl group or carbonyl group at the 12-position is a saturated fatty acid or an unsaturated fatty acid having at least one cis double bond at the 5-position, the 8-position, the 9-position, the 14-position, the 17-position. Also, a saturated fatty acid having 18 carbon atoms and a hydroxyl group at the 12-position is also preferable.

More specifically, the saturated fatty acid or the unsaturated fatty acid having at least one cis double bond at the 6-position, 9-position, the 15-position, which has 18 carbon atoms and a hydroxyl group or carbonyl group at the 13-position, is selected from the group consisting of 13-hydroxy-cis-9-octadecenoic acid, 13-hydroxy-cis-6,cis-9-octadecadienoic acid, 13-hydroxy-cis-9,cis-15-octadecadienoic acid, 13-hydroxy-cis-6,cis-9,cis-15-octadecatrienoic acid, 10,13-dihydroxy-octadecanoic acid, 10,13-dihydroxy-cis-6-octadecenoic acid, 10,13-dihydroxy-cis-15-octadecenoic acid, 10,13-dihydroxy-cis-6,cis-15-octadecadienoic acid, 10-oxo-13-hydroxy-cis-6-octadecenoic acid, 10-oxo-13-hydroxy-cis-15-octadecenoic acid, 10-oxo-13-hydroxy-cis-6,cis-15-octadecadienoic acid, 13-hydroxy-cis-5,cis-9-octadecadienoic acid, 13-hydroxy-trans-5,cis-9-octadecadienoic acid, 13-oxo-cis-9-octadecenoic acid, 13-oxo-cis-6,cis-9-octadecadienoic acid, 13-oxo-cis-9,cis-15-octadecadienoic acid, 13-oxo-cis-6,cis-9,cis-15-octadecatrienoic acid, 10,13-dioxo-octadecanoic acid, 10,13-dioxo-cis-6-octadecenoic acid, 10,13-dioxo-cis-15-octadecenoic acid, 10,13-dioxo-cis-6,cis-15-octadecadienoic acid, 13-oxo-cis-5,cis-9-octadecadienoic acid and 13-oxo-trans-5,cis-9-octadecadienoic acid, preferably, 13-hydroxy-cis-9-octadecenoic acid, 13-hydroxy-cis-6,cis-9-octadecadienoic acid, 13-hydroxy-cis-9,cis-15-octadecadienoic acid, 10,13-dihydroxy-octadecanoic acid, 10,13-dihydroxy-cis-6-octadecenoic acid, 10,13-dihydroxy-cis-15-octadecenoic acid, 13-oxo-cis-9-octadecenoic acid, 13-oxo-cis-9,cis-15-octadecadienoic acid, or 13-oxo-cis-6,cis-9-octadecadienoic acid, more preferably, 13-oxo-cis-9,cis-15-octadecadienoic acid or 13-hydroxy-cis-9,cis-15-octadecadienoic acid.

The saturated fatty acid having 16 carbon atoms and a hydroxyl group or carbonyl group at the 10-position is selected from the group consisting of 10-hydroxy-hexadecanoic acid and 10-oxo-hexadecanoic acid.

The unsaturated fatty acid having at least one cis double bond at the 5-position, the 8-position, the 11-position, the 17-position, which has 20 carbon atoms and a hydroxyl group or carbonyl group at the 15-position, is selected from the group consisting of 15-hydroxy-cis-11-eicosenoic acid, 15-hydroxy-cis-11,cis-17-eicosadienoic acid, 15-hydroxy-cis-8,cis-11-eicosadienoic acid, 15-hydroxy-cis-5,cis-8,cis-11-eicosatrienoic acid, 15-hydroxy-cis-8,cis-11,cis-17-eicosatrienoic acid, 15-hydroxy-cis-5,cis-11-eicosadienoic acid, 15-hydroxy-cis-5,cis-11,cis-17-eicosatrienoic acid, 15-oxo-cis-11-eicosenoic acid, 15-oxo-cis-11,cis-17-eicosadienoic acid, 15-oxo-cis-8,cis-11-eicosadienoic acid, 15-oxo-cis-5,cis-8,cis-11-eicosatrienoic acid, 15-oxo-cis-8,cis-11,cis-17-eicosatrienoic acid, 15-oxo-cis-5,cis-11-eicosadienoic acid and 15-oxo-cis-5,cis-11,cis-17-eicosatrienoic acid.

The a saturated fatty acid or an unsaturated fatty acid having at least one cis double bond at the 5-position, the 8-position, the 9-position, the 14-position, the 17-position, which has 18 or 20 carbon atoms and a hydroxyl group or carbonyl group at the 12-position (preferably, saturated fatty acid having 18 carbon atoms and a hydroxyl group at the 12-position) is selected from the group consisting of 12-hydroxy-cis-14-eicosenoic acid, 12-hydroxy-cis-14,cis-17-eicosadienoic acid, 12-hydroxy-cis-8,cis-14-eicosadienoic acid, 12-hydroxy-cis-5,cis-8-eicosadienoic acid, 12-hydroxy-cis-8,cis-14,cis-17-eicosatrienoic acid, 12-hydroxy-cis-5,cis-8,cis-14-eicosatrienoic acid, 12-oxo-octadecanoic acid, 12-oxo-cis-14-eicosenoic acid, 12-oxo-cis-14,cis-17-eicosadienoic acid, 12-oxo-cis-8,cis-14-eicosadienoic acid, 12-oxo-cis-5,cis-8-eicosadienoic acid, 12-oxo-cis-8,cis-14,cis-17-eicosatrienoic acid, and 12-oxo-cis-5,cis-8,cis-14-eicosatrienoic acid.

The LP-rare fatty acid derivative to be used in the present invention can be prepared by the method of PCT/JP2012/78747 (WO 2013/168310) found by the inventors. In addition, 10-hydroxy-cis-12-octadecenoic acid can be prepared in reference to Biochemical and Biophysical Research Communications 416 (2011) p.188-193 and the like. In addition, the LA-rare fatty acid derivative can be prepared by the following method.

As the LA-rare fatty acid derivative to be used in the present invention, a hydroxylated fatty acid is produced from an unsaturated fatty acid having 16, 18, 20 carbon atoms by a novel fatty acid hydration enzyme (FA-HY), and an oxo fatty acid can be produced by further oxidizing the hydroxyl group of the hydroxylated fatty acid by an enzyme reaction or chemical reaction.

The above-mentioned novel fatty acid hydration enzyme "FA-HY" is
(a) the enzyme protein consisting of the amino acid sequence shown in SEQ ID NO: 2,
(b) a protein comprising an amino acid sequence wherein one or plural amino acids in the amino acid sequence shown in SEQ ID NO: 2 are deleted and/or substituted and/or inserted and/or added, and having an enzyme activity that the enzyme protein consisting of the amino acid sequence shown in SEQ ID NO: 2 has, or
(c) a protein encoded by a base sequence that hybridizes to a nucleic acid consisting of a chain sequence complementary to the base sequence shown in SEQ ID NO: 1 under stringent conditions, and having an enzyme activity that the enzyme protein consisting of the amino acid sequence shown in SEQ ID NO: 2 has.

More specific examples of the above-mentioned (b) include a protein containing (i) an amino acid sequence which is the amino acid sequence shown in SEQ ID NO: 2, wherein 1 - 20, preferably 1 - 10, more preferably 1 - several (5, 4, 3 or 2) amino acids are deleted, (ii) an amino acid sequence which is the amino acid sequence shown in SEQ ID NO: 2, wherein 1 - 20, preferably 1 - 10, more preferably 1 - several number (5, 4, 3 or 2) amino acids are added, (iii) an amino acid sequence which is the amino acid sequence shown in SEQ ID NO: 2, wherein 1 - 20, preferably 1 - 10, more preferably 1 - several (5, 4, 3 or 2) amino acids are inserted, (iv) an amino acid sequence which is the amino acid sequence shown in SEQ ID NO: 2, wherein 1 - 20, preferably 1 - 10, more preferably 1 - several (5, 4, 3 or 2) amino acids are substituted by other amino acids, or (v) an amino acid sequence obtained by combining them. When amino acids with similar properties (e.g., glycine and alanine, valine and leucine and isoleucine, serine and threonine, aspartic acid and glutamic acid, asparagine and glutamine, lysine and arginine, cysteine and methionine, phenylalanine and tyrosine etc.) are substituted with each other and the like, a greater number of substitutions and the like are possible.

When amino acids are deleted, substituted or inserted as mentioned above, the positions of deletion, substitution and insertion are not particularly limited as long as the above-mentioned enzyme activity is maintained.

In the above-mentioned (c), the "stringent conditions" are conditions under which nucleotide sequences having high identity, for example, identity of 70, 80, 90, 95 or 99% or above, hybridize to each other and nucleotide sequences having identity lower than that do not hybridize; specifically, conditions of washing once, more preferably 2 - 3 times, at the salt concentration and temperature corresponding to those in the washing conditions of general Southern hybridization (60°C, 1xSSC, 0.1% SDS, preferably, 0.1xSSC, 0.1% SDS, more preferably, 68°C, 0.1xSSC, 0.1% SDS) and the like.

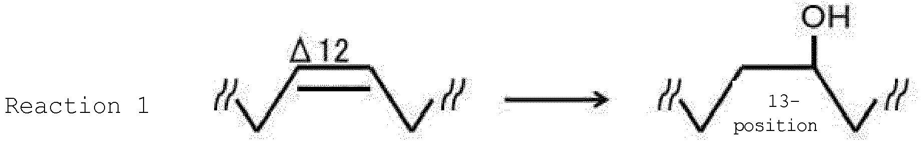

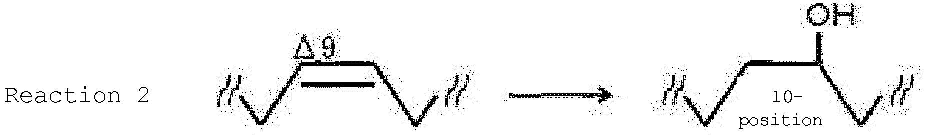

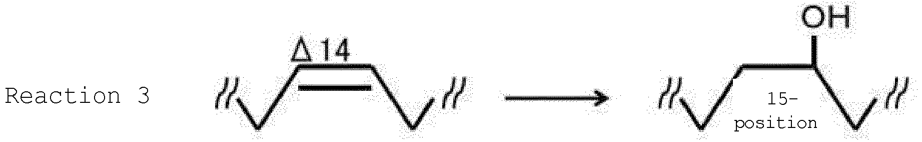

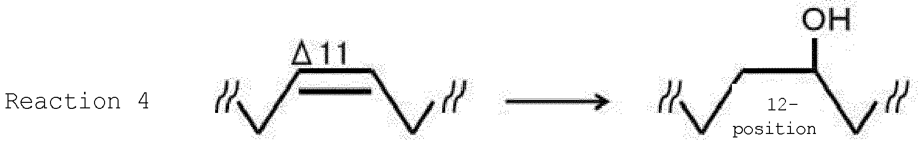

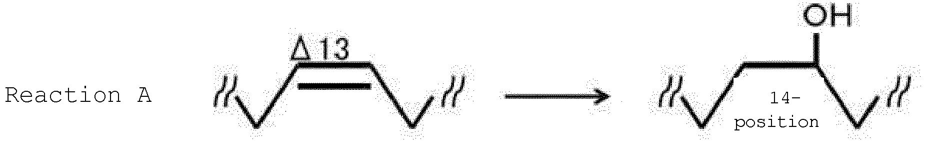

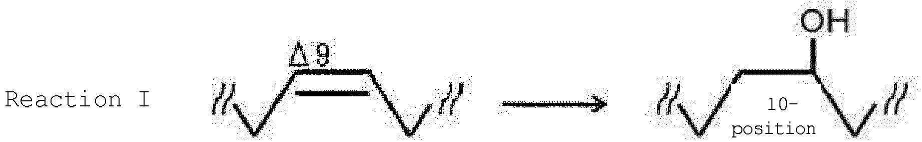

Regarding the above-mentioned (b) or (c), the enzyme activity that the enzyme protein consisting of the amino acid sequence shown in SEQ ID NO: 2 has is not particularly limited as long as it has at least one, preferably all, of (1) an enzyme activity capable of converting an unsaturated fatty acid having 18 carbon atoms and a cis double bond at the 12-position (hereinafter sometimes to be abbreviated as "cis-12 unsaturated fatty acid") utilized as a substrate to a hydroxylated fatty acid having 18 carbon atoms and a hydroxyl group at the 13-position (13-hydroxy fatty acid) (reaction 1), (2) an enzyme activity capable of converting an unsaturated fatty acid having 16 carbon atoms and a cis double bond at the 9-position (hereinafter sometimes to be abbreviated as "cis-9 unsaturated fatty acid")) utilized as a substrate to a hydroxylated fatty acid having 16 carbon atoms and a hydroxyl group at the 10-position (10-hydroxy fatty acid) (reaction 2), (3) an enzyme activity capable of converting an unsaturated fatty acid having 20 carbon atoms and a cis double bond at the 14-position (hereinafter sometimes to be abbreviated as "cis-14 unsaturated fatty acid")) utilized as a substrate to a hydroxylated fatty acid having 20 carbon atoms and a hydroxyl group at the 15-position (15-hydroxy fatty acid) (reaction 3), (4) an enzyme activity capable of converting an unsaturated fatty acid having 18 or 20 carbon atoms and a cis double bond at the 11-position (hereinafter sometimes to be abbreviated as "cis-11 unsaturated fatty acid")) utilized as a substrate to a hydroxylated fatty acid having 18 or 20 carbon atoms and a hydroxyl group at the 12-position (12-hydroxy fatty acid) (reaction 4), an enzyme activity capable of converting cis-4,cis-7,cis-10,cis-13,cis-16,cis-19-docosahexaenoic acid (DHA) to 14-hydroxy-cis-4,cis-7,cis-10,cis-16,cis-19-docosapentaenoic acid (reaction A), and an enzyme activity capable of converting cis-9-tetradecenoic acid (myristoleic acid) to 10-hydroxy-tetradecanoic acid (reaction I).

The above-mentioned "cis-12 unsaturated fatty acid", "cis-9 unsaturated fatty acid", "cis-14 unsaturated fatty acid", and "cis-11 unsaturated fatty acid" are not particularly limited as long as they are an unsaturated fatty acid having 18 carbon atoms and a cis double bond at the 12-position, an unsaturated fatty acid having 16 carbon atoms and a cis double bond at the 9-position, an unsaturated fatty acid having 20 carbon atoms and a cis double bond at the 14-position, an unsaturated fatty acid having 18 or 20 carbon atoms and a cis double bond at the 11-position, respectively and, for example, monovalent unsaturated fatty acid, divalent unsaturated fatty acid, trivalent unsaturated fatty acid, tetravalent unsaturated fatty acid, pentavalent unsaturated fatty acid and the like can be mentioned. In the present specification, the "fatty acid" encompasses not only free acids but also ester form, salt with basic compound and the like. The "DHA" and "myristoleic acid" also encompass not only free acids but also ester form, salt with basic compound and the like.

The above-mentioned FA-HY can be isolated from, for example, the fungus, culture medium of Lactobacillus acidophilus by a protein separation and purification technique known per se. Alternatively, FA-HY may be used as the fungus of Lactobacillus acidophilus containing FA-HY or fungal debris thereof. The fungus of Lactobacillus acidophilus containing FA-HY is not particularly limited as long as it contains the above-mentioned FA-HY and, for example, NITE BP-01788 deposited on January 17, 2014 at the NITE Patent Microorganisms Depositary (NPMD) and the like can be mentioned. Alternatively, FA-HY can also be produced as a recombinant protein by isolating a gene encoding FA-HY, subcloning same into a suitable vector, introducing same into a suitable host such as Escherichia coli and the like and culturing same. FA-HY may be a purified one or a crudely purified one. Alternatively, hydratase may be expressed in fungus such as Escherichia coli and the like and the fungus itself may be used or culture medium thereof may be used. Furthermore, the enzyme may be of a free form, or immobilized by various carriers.

As a vector containing a nucleic acid encoding the above-mentioned FA-HY, one suitable for a host cell to be introduced with the vector may be appropriately selected according to the object (e.g., protein expression) and can be used. In the case of an expression vector, it contains the nucleic acid of the present invention, which is operably linked to an appropriate promoter, and preferably contains a transcription termination signal, i.e., terminator region, at the downstream of the nucleic acid of the present invention. Furthermore, it can also contain a selection marker gene for selection of a transformant (drug resistance gene, gene that complements auxotrophic mutation etc.). Also, it may contain a sequence encoding a tag sequence useful for separation and purification of the expressed protein and the like. In addition, the vector may be incorporated into the genome of a target host cell. The vector of the present invention can be introduced into a target host cell by a transformation method known per se such as a competent cell method, a protoplast method, a calcium phosphate coprecipitation method and the like.

In the present specification, the "host cell" may be any cell as long as it can express a vector containing a nucleic acid encoding the above-mentioned FA-HY, and bacterium, yeast, fungi, higher eukaryotic cell and the like can be mentioned. Examples of the bacterium include gram-positive bacteria such as bacillus, Streptomyces and the like and gram negative bacteria such as Escherichia coli and the like. A recombinant cell introduced with a vector containing a nucleic acid encoding FA-HY can be cultivated by a method known per se which is suitable for the host cell.

"Purification" of the above-mentioned FA-HY can be performed by a method known per se, for example, fungi collected by centrifugation and the like are ruptured by ultrasonication or glass beads and the like, solid such as cell debris is removed by centrifugation and the like, and the like to give a crude enzyme solution, which is subjected to a salting out method using ammonium sulfate, sodium sulfate and the like, chromatographys such as ion exchange chromatography, gel filtration chromatography, affinity chromatography and the like, gel electrophoresis and the like.

The above-mentioned FA-HY has, as mentioned above, an enzyme activity capable of converting cis-12 unsaturated fatty acid, cis-9 unsaturated fatty acid, cis-14 unsaturated fatty acid, cis-11 unsaturated fatty acid, DHA, myristoleic acid utilized as substrates to 13-hydroxy fatty acid, 10-hydroxy fatty acid, 15-hydroxy fatty acid, 12-hydroxy fatty acid, 14-hydroxy-cis-4,cis-7,cis-10,cis-16,cis-19-docosapentaenoic acid, 10-hydroxy-tetradecanoic acid, respectively. Therefore, the present disclosure also provides [1] a method of producing 13-hydroxy fatty acid from cis-12 unsaturated fatty acid by a hydration reaction using the above-mentioned FA-HY (production method 1), [2] a method of producing 10-hydroxy fatty acid from cis-9 unsaturated fatty acid by a hydration reaction using the above-mentioned FA-HY (production method 2), [3] a method of producing 15-hydroxy fatty acid from cis-14 unsaturated fatty acid by a hydration reaction using the above-mentioned FA-HY (production method 3), [4] a method of producing 12-hydroxy fatty acid from cis-11 unsaturated fatty acid by a hydration reaction using the above-mentioned FA-HY (production method 4), [A] a method of producing 14-hydroxy-cis-4,cis-7,cis-10,cis-16,cis-19-docosapentaenoic acid from DHA by a hydration reaction using the above-mentioned FA-HY (production method A), and [I] a method of producing 10-hydroxy-tetradecanoic acid from myristoleic acid by a hydration reaction using the FA-HY of the present invention (production method I).

Examples of the "cis-12 unsaturated fatty acid" in the above-mentioned production method 1 include cis-9,cis-12-octadecadienoic acid (linoleic acid), cis-6,cis-9,cis-12-octadecatrienoic acid (γ-linolenic acid), cis-9,cis-12,cis-15-octadecatrienoic acid (α-linolenic acid), cis-6,cis-9,cis-12,cis-15-octadecatetraenoic acid (stearidonic acid), as well as 10-hydroxy-cis-12-octadecenoic acid, 10-hydroxy-cis-6,cis-12-octadecadienoic acid, 10-hydroxy-cis-12,cis-15-octadecadienoic acid, 10-hydroxy-cis-6,cis-12,cis-15-octadecatrienoic acid, 10-oxo-cis-12-octadecenoic acid, 10-oxo-cis-6,cis-12-octadecadienoic acid, 10-oxo-cis-12,cis-15-octadecadienoic acid, 10-oxo-cis-6,cis-12,cis-15-octadecatrienoic acid, cis-5,cis-9,cis-12-octadecatrienoic acid(pinolenic acid), trans-5,cis-9,cis-12-octadecatrienoic acid (columbinic acid), which are now producible by WO 2013/168310, and the like. These substrates may be obtained by a method other than WO 2013/168310.

Examples of the "13-hydroxy fatty acid" produced by the above-mentioned production method 1 include 13-hydroxy-cis-9-octadecenoic acid induced from cis-9,cis-12-octadecadienoic acid (linoleic acid), 13-hydroxy-cis-6,cis-9-octadecadienoic acid induced from cis-6,cis-9,cis-12-octadecatrienoic acid (γ-linolenic acid), 13-hydroxy-cis-9,cis-15-octadecadienoic acid induced from cis-9,cis-12,cis-15-octadecatrienoic acid (α-linolenic acid), 13-hydroxy-cis-6,cis-9,cis-15-octadecatrienoic acid induced from cis-6,cis-9,cis-12,cis-15-octadecatetraenoic acid (stearidonic acid), 10,13-dihydroxy-octadecanoic acid induced from 10-hydroxy-cis-12-octadecenoic acid, 10,13-dihydroxy-cis-6-octadecenoic acid induced from 10-hydroxy-cis-6,cis-12-octadecadienoic acid, 10,13-dihydroxy-cis-15-octadecenoic acid induced from 10-hydroxy-cis-12,cis-15-octadecadienoic acid, 10,13-dihydroxy-cis-6,cis-15-octadecadienoic acid induced from 10-hydroxy-cis-6,cis-12,cis-15-octadecatrienoic acid, 10-oxo-13-hydroxy-cis-6-octadecenoic acid induced from 10-oxo-cis-6,cis-12-octadecadienoic acid, 10-oxo-13-hydroxy-cis-15-octadecenoic acid induced from 10-oxo-cis-12,cis-15-octadecadienoic acid, 10-oxo-13-hydroxy-cis-6,cis-15-octadecadienoic acid induced from 10-oxo-cis-6,cis-12,cis-15-octadecatrienoic acid, 13-hydroxy-cis-5,cis-9-octadecadienoic acid induced from cis-5,cis-9,cis-12-octadecatrienoic acid (pinolenic acid), 13-hydroxy-trans-5,cis-9-octadecadienoic acid induced from trans-5,cis-9,cis-12-octadecatrienoic acid (columbinic acid) and the like.

Examples of the "cis-9 unsaturated fatty acid" in the above-mentioned production method 2 include cis-9-hexadecenoic acid (pulmitoleic acid) and the like.

Examples of the "10-hydroxy fatty acid" produced by the above-mentioned production method 2 include 10-hydroxy-hexadecanoic acid induced from cis-9-hexadecenoic acid (pulmitoleic acid) and the like.

Examples of the "cis-14 unsaturated fatty acid" in the above-mentioned production method 3 include cis-11,cis-14-eicosadienoic acid, cis-11,cis-14,cis-17-eicosatrienoic acid, cis-8,cis-11,cis-14-eicosatrienoic acid (dihomo-γ-linolenic acid), cis-5,cis-8,cis-11,cis-14-eicosatetraenoic acid (arachidonic acid), cis-8,cis-11,cis-14,cis-17-eicosatetraenoic acid, cis-5,cis-11,cis-14-eicosatrienoic acid (sciadonic acid), cis-5,cis-11,cis-14,cis-17-eicosatetraenoic acid (juniperonic acid) and the like.

Examples of the "15-hydroxy fatty acid" produced by the above-mentioned production method 3 include 15-hydroxy-cis-11-eicosenoic acid induced from cis-11,cis-14-eicosadienoic acid, 15-hydroxy-cis-11,cis-17-eicosadienoic acid induced from cis-11,cis-14,cis-17-eicosatrienoic acid, 15-hydroxy-cis-8,cis-11-eicosadienoic acid induced from cis-8,cis-11,cis-14-eicosatrienoic acid (dihomo-γ-linolenic acid), 15-hydroxy-cis-5,cis-8,cis-11-eicosatrienoic acid induced from cis-5,cis-8,cis-11,cis-14-eicosatetraenoic acid (arachidonic acid), 15-hydroxy-cis-8,cis-11,cis-17-eicosatrienoic acid induced from cis-8,cis-11,cis-14,cis-17-eicosatetraenoic acid, 15-hydroxy-cis-5,cis-11-eicosadienoic acid induced from cis-5,cis-11,cis-14-eicosatrienoic acid (sciadonic acid), 15-hydroxy-cis-5,cis-11,cis-17-eicosatrienoic acid induced from cis-5,cis-11,cis-14,cis-17-eicosatetraenoic acid (juniperonic acid) and the like.

Examples of the "cis-11 unsaturated fatty acid" in the above-mentioned production method 4 include cis-11-octadecenoic acid (cis-vaccenic acid), cis-11,cis-14-eicosadienoic acid, cis-11,cis-14,cis-17-eicosatrienoic acid, cis-8,cis-11,cis-14-eicosatrienoic acid (dihomo-γ-linolenic acid), cis-5,cis-8,cis-11-eicosatrienoic acid (mead acid), cis-8,cis-11,cis-14,cis-17-eicosatetraenoic acid, cis-5,cis-8,cis-11,cis-14-eicosatetraenoic acid (arachidonic acid) and the like.

Examples of the "12-hydroxy fatty acid" produced by the above-mentioned production method 4 include 12-hydroxy-octadecanoic acid induced from cis-11-octadecenoic acid(cis-vaccenic acid), 12-hydroxy-cis-14-eicosenoic acid induced from cis-11,cis-14-eicosadienoic acid, 12-hydroxy-cis-14,cis-17-eicosadienoic acid induced from cis-11,cis-14,cis-17-eicosatrienoic acid, 12-hydroxy-cis-8,cis-14-eicosadienoic acid induced from cis-8,cis-11,cis-14-eicosatrienoic acid (dihomo-γ-linolenic acid), 12-hydroxy-cis-5,cis-8-eicosadienoic acid induced from cis-5,cis-8,cis-11-eicosatrienoic acid (mead acid), 12-hydroxy-cis-8,cis-14,cis-17-eicosatrienoic acid induced from cis-8,cis-11,cis-14,cis-17-eicosatetraenoic acid, 12-hydroxy-cis-5,cis-8,cis-14-eicosatrienoic acid induced from cis-5,cis-8,cis-11,cis-14-eicosatetraenoic acid (arachidonic acid) and the like.

The hydration reaction may be performed in a suitable buffer (e.g., phosphate buffer, tris buffer, borate buffer etc.) by mixing unsaturated fatty acid, which is a substrate, and the above-mentioned FA-HY at suitable concentrations and incubating the mixture. The substrate concentration is, for example, 1 - 1000 g/L, preferably 10 - 500 g/L, more preferably 20 - 250 g/L. The amount of the above-mentioned FA-HY to be added is, for example, 0.001 - 10 mg/mL, preferably 0.1 - 5 mg/mL, more preferably 0.2 - 2 mg/mL.

A "cofactor" may be used for a hydration reaction (reaction 1 - 4, reaction A or reaction I) and, for example, FAD and the like can be used. The concentration of addition may be any as long as the hydration reaction proceeds efficiently. It is preferably 0.001 - 20 mM, more preferably 0.01 - 10 mM.

Furthermore, an "activator" may be used for the hydration reaction and, for example, 1 or 2 compounds selected from the group consisting of NADH and NADPH can be mentioned. The concentration of addition thereof may be any as long as the hydration reaction proceeds efficiently. It is preferably 0.1 - 20 mM, more preferably 1 - 10 mM.

The hydration reaction is desirably performed at a preferable temperature and in a preferable pH range for the above-mentioned FA-HY. For example, the reaction temperature is 5 - 50°C, preferably 20 - 45°C. The pH of the reaction mixture is, for example, pH 4 - 10, preferably pH 5 - 9. The reaction time is not particularly limited and it is, for example, 10 min - 72 hr, preferably 30 min - 36 hr.

In one preferable embodiment of the present invention, the above-mentioned FA-HY is provided to the reaction system in the form of recombinant cells (e.g., Escherichia coli, Bacillus subtilis, yeast, insect cell, animal cell etc.) introduced with an expression vector containing a nucleic acid encoding same. In this case, the hydration reaction can also be performed by cultivating the cells in a liquid medium suitable for the culture of the cells and added with cofactor and a substrate and, where necessary, an activator.

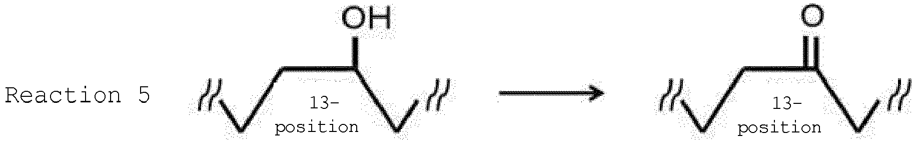

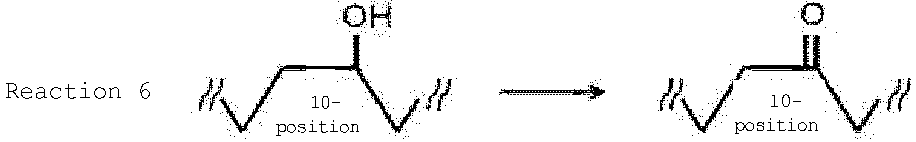

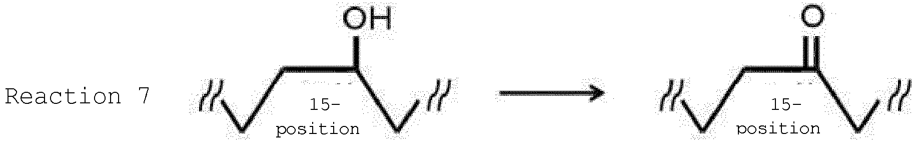

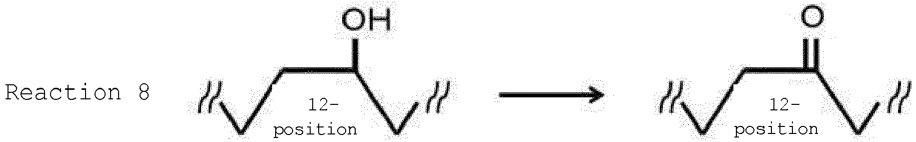

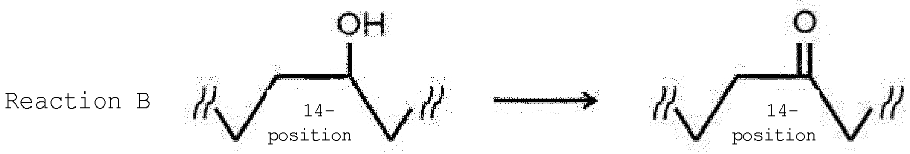

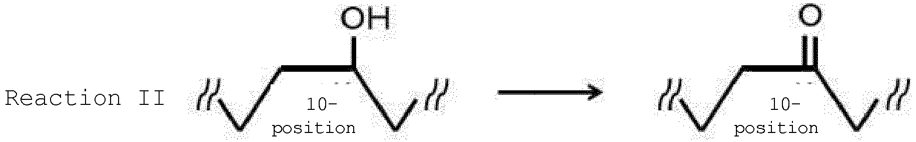

Furthermore, by a dehydrogenation reaction or chemical oxidation using chrome acid, an oxo fatty acid having 18 carbon atoms and a carbonyl group at the 13-position (hereinafter sometimes to be abbreviated as "13-oxo fatty acid") is produced from 13-hydroxy fatty acid obtained in the above-mentioned production methods 1 - 4, production method A, production method I (reaction 5), an oxo fatty acid having 16 carbon atoms and a carbonyl group at the 10-position (hereinafter sometimes to be abbreviated as "10-oxo fatty acid") is produced from 10-hydroxy fatty acid (reaction 6), an oxo fatty acid having 20 carbon atoms and a carbonyl group at the 15-position (hereinafter sometimes to be abbreviated as "15-oxo fatty acid") is produced from 15-hydroxy fatty acid (reaction 7), an oxo fatty acid having 18 or 20 carbon atoms and a carbonyl group at the 12-position (hereinafter sometimes to be abbreviated as "12-oxo fatty acid") is produced from 12-hydroxy fatty acid (reaction 8), 14-oxo-cis-4,cis-7,cis-10,cis-16,cis-19-docosapentaenoic acid is produced from 14-hydroxy-cis-4,cis-7,cis-10,cis-16,cis-19-docosapentaenoic acid (reaction B), and 10-oxo-tetradecanoic acid is produced from 10-hydroxy-tetradecanoic acid (reaction II).

Therefore, the present disclosure also provides [5] a method of producing 13-oxo fatty acid, comprising subjecting cis-12 unsaturated fatty acid to a hydration reaction using the above-mentioned FA-HY to induce 13-hydroxy fatty acid, and subjecting the 13-hydroxy fatty acid to a dehydrogenation reaction or chemical oxidation (production method 5), [6] a method of producing 10-oxo fatty acid, comprising subjecting cis-9 unsaturated fatty acid to a hydration reaction using the above-mentioned FA-HY to induce 10-hydroxy fatty acid, and subjecting the 10-hydroxy fatty acid to a dehydrogenation reaction or chemical oxidation (production method 6), [7] a method of producing 15-oxo fatty acid, comprising subjecting cis-14 unsaturated fatty acid to a hydration reaction using the above-mentioned FA-HY to induce 15-hydroxy fatty acid, and subjecting the 15-hydroxy fatty acid to a dehydrogenation reaction or chemical oxidation (production method 7), [8] a method of producing 12-oxo fatty acid, comprising subjecting cis-11 unsaturated fatty acid to a hydration reaction using the above-mentioned FA-HY to induce 12-hydroxy fatty acid, and subjecting the 12-hydroxy fatty acid to a dehydrogenation reaction or chemical oxidation (production method 8), [B] a method of producing 14-oxo-cis-4,cis-7,cis-10,cis-16,cis-19-docosapentaenoic acid, comprising subjecting DHA to a hydration reaction using the FA-HY of the present invention to induce 14-hydroxy-cis-4,cis-7,cis-10,cis-16,cis-19-docosapentaenoic acid, and subjecting the 14-hydroxy-cis-4,cis-7,cis-10,cis-16,cis-19-docosapentaenoic acid to a dehydrogenation reaction or chemical oxidation (production method B), and [II] a method of producing 10-oxo-tetradecanoic acid, comprising subjecting myristoleic acid to a hydration reaction using the FA-HY of the present invention to induce 10-hydroxy-tetradecanoic acid, and subjecting the 10-hydroxy-tetradecanoic acid to a dehydrogenation reaction or chemical oxidation (production method II).

The "cis-12 unsaturated fatty acid", "cis-9 unsaturated fatty acid", "cis-14 unsaturated fatty acid", "cis-11 unsaturated fatty acid" in the above-mentioned production methods 5 - 8 are the same as the substrates in the above-mentioned production methods 1 - 4.

Examples of the "13-oxo fatty acid" produced by the above-mentioned production method 5 include 13-oxo-cis-9-octadecenoic acid induced from cis-9,cis-12-octadecadienoic acid (linoleic acid), 13-oxo-cis-6,cis-9-octadecadienoic acid induced from cis-6,cis-9,cis-12-octadecatrienoic acid (γ-linolenic acid), 13-oxo-cis-9,cis-15-octadecadienoic acid induced from cis-9,cis-12,cis-15-octadecatrienoic acid (α-linolenic acid), 13-oxo-cis-6,cis-9,cis-15-octadecatrienoic acid induced from cis-6,cis-9,cis-12,cis-15-octadecatetraenoic acid (stearidonic acid), 10,13-dioxo-octadecanoic acid induced from 10-hydroxy-cis-12-octadecenoic acid or 10-oxo-cis-12-octadecenoic acid, 10,13-dioxo-cis-6-octadecenoic acid induced from 10-hydroxy-cis-6,cis-12-octadecadienoic acid or 10-oxo-cis-6,cis-12-octadecadienoic acid, 10,13-dioxo-cis-15-octadecenoic acid induced from 10-hydroxy-cis-12,cis-15-octadecadienoic acid or 10-oxo-cis-12,cis-15-octadecadienoic acid, 10,13-dioxo-cis-6,cis-15-octadecadienoic acid induced from 10-hydroxy-cis-6,cis-12,cis-15-octadecatrienoic acid or 10-oxo-cis-6,cis-12,cis-15-octadecatrienoic acid, 13-oxo-cis-5,cis-9-octadecadienoic acid induced from cis-5,cis-9,cis-12-octadecatrienoic acid (pinolenic acid), 13-oxo-trans-5,cis-9-octadecadienoic acid induced from trans-5,cis-9,cis-12-octadecatrienoic acid (columbinic acid) and the like.

Examples of the "10-oxo fatty acid" produced by the above-mentioned production method 6 include 10-oxo-hexadecanoic acid induced from cis-9-hexadecenoic acid (pulmitoleic acid) and the like.

Examples of the "15-oxo fatty acid" produced by the above-mentioned production method 7 include15-oxo-cis-11-eicosenoic acid induced from cis-11,cis-14-eicosadienoic acid, 15-oxo-cis-11,cis-17-eicosadienoic acid induced from cis-11,cis-14,cis-17-eicosatrienoic acid, 15-oxo-cis-8,cis-11-eicosadienoic acid induced from cis-8,cis-11,cis-14-eicosatrienoic acid (dihomo-γ-linolenic acid), 15-oxo-cis-5,cis-8,cis-11-eicosatrienoic acid induced from cis-5,cis-8,cis-11,cis-14-eicosatetraenoic acid (arachidonic acid), 15-oxo-cis-8,cis-11,cis-17-eicosatrienoic acid induced from cis-8,cis-11,cis-14,cis-17-eicosatetraenoic acid, 15-oxo-cis-5,cis-11-eicosadienoic acid induced from cis-5,cis-11,cis-14-eicosatrienoic acid (sciadonic acid), 15-oxo-cis-5,cis-11,cis-17-eicosatrienoic acid induced from cis-5,cis-11,cis-14,cis-17-eicosatetraenoic acid (juniperonic acid) and the like.

Examples of the "12-oxo fatty acid" produced by the above-mentioned production method 8 include 12-oxo-octadecanoic acid induced from cis-11-octadecenoic acid (cis-vaccenic acid), 12-oxo-cis-14-eicosenoic acid induced from cis-11,cis-14-eicosadienoic acid, 12-oxo-cis-14,cis-17-eicosadienoic acid induced from cis-11,cis-14,cis-17-eicosatrienoic acid, 12-oxo-cis-8,cis-14-eicosadienoic acid induced from cis-8,cis-11,cis-14-eicosatrienoic acid (dihomo-γ-linolenic acid), 12-oxo-cis-5,cis-8-eicosadienoic acid induced from cis-5,cis-8,cis-11-eicosatrienoic acid (mead acid), 12-oxo-cis-8,cis-14,cis-17-eicosatrienoic acid induced from cis-8,cis-11,cis-14,cis-17-eicosatetraenoic acid, 12-oxo-cis-5,cis-8,cis-14-eicosatrienoic acid induced from cis-5,cis-8,cis-11,cis-14-eicosatetraenoic acid (arachidonic acid) and the like.

The dehydrogenase to be used in the above-mentioned production methods 5 - 8, production method B or production method II is not particularly limited as long as it is an enzyme capable of converting 13-hydroxy fatty acid, 10-hydroxy fatty acid, 15-hydroxy fatty acid, 12-hydroxy fatty acid, 14-hydroxy-cis-4,cis-7,cis-10,cis-16,cis-19-docosapentaenoic acid, 10-hydroxy-tetradecanoic acid utilized as substrates to 13-oxo fatty acid, 10-oxo fatty acid, 15-oxo fatty acid, 12-oxo fatty acid, 14-oxo-cis-4,cis-7,cis-10,cis-16,cis-19-docosapentaenoic acid, 10-oxo-tetradecanoic acid, respectively and, for example, lactobacillus-derived hydroxylated fatty acid - dehydrogenase (CLA-DH) is preferable. More preferred is Lactobacillus plantarum-derived CLA-DH, and particularly preferred is L. plantarum FERM BP-10549 strain-derived CLA-DH. CLA-DH can be obtained by the method described in JP-A-2007-259712, the method described in WO 2013/168310. Dehydrogenase may be a purified one or a crudely purified one. Alternatively, dehydrogenase may be expressed in fungus such as Escherichia coli and the like and the fungus itself may be used or culture medium thereof may be used. Furthermore, the enzyme may be of a free form, or immobilized by various carriers.

The dehydrogenation reaction is performed in a suitable buffer (e.g., phosphate buffer, tris buffer, borate buffer etc.) by mixing 13-hydroxy fatty acid, 10-hydroxy fatty acid, 15-hydroxy fatty acid, 12-hydroxy fatty acid, as substrates and dehydrogenase at suitable concentrations and incubating the mixture. The substrate concentration is, for example, 0.01 - 100 g/L, preferably 0.05 - 50 g/L, more preferably 0.1 - 5 g/L. The amount of dehydrogenase to be added is, for example, 0.001 - 10 mg/mL, preferably 0.005 - 1 mg/mL, more preferably 0.05 - 0.2 mg/mL.

A "cofactor" may be used for the dehydrogenation reaction and, for example, NAD⁺, NADP⁺ and the like can be used. The concentration of addition thereof may be any as long as the hydration reaction proceeds efficiently. It is preferably 0.001 - 20 mM, more preferably 0.01 - 10 mM.

The dehydrogenation reaction is desirably performed within the ranges of preferable temperature and preferable pH of dehydrogenase. For example, the reaction temperature is 5 - 50°C, preferably 20 - 45°C. The pH of the reaction mixture is, for example, pH 4 - 10, preferably pH 5 - 9. The reaction time is not particularly limited and it is, for example, 10 min - 72 hr, preferably 30 min - 36 hr.

In one embodiment, dehydrogenase is subjected to the reaction system in the form of recombinant cells (e.g., Escherichia coli, Bacillus subtilis, yeast, insect cell, animal cell etc.) introduced with an expression vector containing a nucleic acid encoding same. In this case, the oxidation reaction can also be performed by cultivating the cells in a liquid medium suitable for the culture of the cells and added with a substrate and, where necessary, a cofactor and an activator.

In addition, by replacing the dehydrogenation reaction with a chemical oxidation using chromic acid, an oxo fatty acid similar to that by enzyme reaction can be chemically obtained.

As the chemical oxidation, methods known per se, for example, chromic acid oxidation, preferably Jones oxidation and the like can be mentioned. As the chromic acid, salts or complexes of the compound such as anhydrous chromic acid CrO₃, chromic acid H₂CrO₄ and dichromic acid H₂Cr₂O₇ can be used.

To be specific, sulfuric acid (2.3 ml) and water (7.7 ml) are added to anhydrous chromic acid (2.67 g), and acetone (90 ml) is added to the mixture to give a chromic acid solution. 2 g of hydroxylated fatty acid and 40 ml of acetone are added in an Erlenmeyer flask, and the above-mentioned chromic acid solution is added by one drop while stirring in a stirrer on ice. When the solution turns from blue to tea green, dropwise addition of the chromic acid solution is stopped, and the reaction is discontinued with isopropyl alcohol. The precipitated sediment is filtered through filter paper, placed in a separating funnel, diethyl ether (150 ml) and Milli-Q water (300 ml) are added, the mixture is shaken well, and the diethyl ether layer is washed several times with Milli-Q water. To the diethyl ether layer after washing is added an appropriate amount of sodium sulfate (anhydrous), the mixture is stirred and the residual water is removed. The anhydrous sodium sulfate added is filtered off through filter paper, the obtained diethyl ether layer is concentrated by a rotary evaporator, and the reaction product (oxo fatty acid) and unreacted substrate are extracted.

An extract obtained by an oxidation reaction with anhydrous chromic acid (mixture containing substrate and resultant product (oxo fatty acid)) is subjected to moderate-pressure chromatography, a solution that comes out from the column is recovered in fractions. The recovered each fraction is analyzed by LC/MS and gas chromatography, fractions containing oxo fatty acid alone are collected and concentrated by a rotary evaporator. A part of the obtained final resultant product is methylesterified, the purity of oxo fatty acid is evaluated by gas chromatography, and oxo fatty acid having a purity of not less than 98% can be obtained.

An anti-inflammatory agent containing the rare fatty acid derivative of the present invention is for use in the prophylaxis or improvement of inflammatory diseases. The "inflammatory disease" is not limited as long as it accompanies an inflammation reaction in vivo, and an "inflammatory disease involving macrophage" is preferable. Examples of the "inflammatory disease" include gout, arteriosclerosis, gastric ulcer, nephritis (glomerulonephritis, IgA nephropathy, diabetic nephropathy etc.), periodontal disease (gingiva inflammation, pericoronitis etc.), hepatitis (alcoholic hepatitis, non-alcoholic hepatitis etc.), cirrhosis, asthma, bronchitis, cerebral infarction, aneurysm, delayed allergy, endometriosis, acute respiratory distress syndrome, disorder due to kidney transplantation, acute myocardial infarction, diabetes, systemic lupus erythematosus, Crohn's disease, atopic dermatitis, pneumonia, arthritis (chronic rheumatoid-like arthritis etc.), endotoxin shock, sepsis due to infections, chronic ulcerative colitis, chronic bronchitis, cystitis, chronic osteomyelitis, erosive esophagitis, cholangitis, chronic cholecystitis, gastritis, chronic cervix inflammation, the following nerve inflammation disease, cancer caused by inflammation and the like. Of these, the "inflammatory disease involving macrophage" includes, for example, hepatitis, asthma, systemic lupus erythematosus, Crohn's disease, atopic dermatitis, arthritis, diabetic neuropathy, and dementia.

Furthermore, an anti-inflammatory agent containing the rare fatty acid derivative as described herein can also be applied to the prophylaxis or improvement of nerve inflammation diseases. The "nerve inflammation disease" refers to a disease caused by nerval inflammation, and the agent can also be used for the prophylaxis or improvement of diabetic neuropathy, dementia (Alzheimer-type etc.), multiple sclerosis and the like. Alternatively, an anti-inflammatory agent containing the rare fatty acid derivative as described herein can also be applied to the prophylaxis or improvement of cancer caused by inflammation. The "cancer caused by inflammation" is a cancer resulting from chronic inflammation, and the agent can also be used for the prophylaxis or improvement of, for example, large intestine cancer, lung cancer, bladder cancer, mouth cavity cancer, tongue cancer, skin cancer, esophageal cancer, melanoma, bile duct cancer, large intestine cancer, gall bladder cancer, stomach cancer, cervix cancer, liver cancer and the like.

In addition, the rare fatty acid derivative as described herein can also be used as an inhibitor of M1 macrophage. Activated macrophage becomes M1 macrophage, produces inflammatory mediators such as nitric oxide (NO), prostaglandin E2, TNF-α and the like, and causes chronic inflammations. As shown in the below-mentioned Examples, the rare fatty acid derivative of the present invention has an effect to suppress NO production amount by M1 macrophage, and induce differentiation of macrophage and monocyte, which is a progenitor cell thereof, into M2 macrophage. Differentiation into M2 macrophage can also be induced by a direct action on monocyte or macrophage before differentiation, as well as by an indirect action obtained by placing the intestine in a Th2 cytokine-dominant environment. Therefore, inflammatory diseases involving macrophage can be prevented or improved by suppression of NO production by M1 macrophage, and suppression of differentiation into M1 macrophage by promoting differentiation of macrophage and monocyte into M2 macrophage.

Furthermore, the rare fatty acid derivative as described herein can also be used as an inhibitor of cell death based on the oxidative stress. The oxidative stress is a stress caused by active oxygen, and examples of the active oxygen include H₂O₂, superoxide, hydroxy radical, oxygen and the like. The active oxygen promotes production of inflammatory cytokines and causes inflammatory diseases. Therefore, the inflammatory diseases can be prevented or improved by reducing the oxidative stress.

Alternatively, the rare fatty acid derivative as described herein can also be used as a promoter of HO-1 expression. HO-1 is an important enzyme as a defense mechanism in the body that protects cells from oxidative stress. Lack of HO-1 promotes cell injury due to oxidative stress and promotes inflammation. Therefore, inflammatory diseases can be prevented or improved by promoting HO-1 expression.

In addition, the rare fatty acid derivative as described herein can also be used as a promoter of intranuclear expression of Nrf2. When intranuclear expression of Nrf2 is promoted, Nrf2 controls oxidative stress-adoptive reaction and suppresses inflammation. Therefore, inflammatory diseases can be prevented or improved by promoting intranuclear expression of Nrf2.

An anti-inflammatory agent (or M1 macrophage inhibitor, inhibitor of cell death based on oxidative stress, HO-1 expression promoter or Nrf2 intranuclear expression promoter) containing the rare fatty acid derivative for use according to the present invention can be, for example, a pharmaceutical product, a food, a feed, a cosmetic and the like, or the agent may be added to them.

The dosage form of the pharmaceutical product includes dispersion, granule, pill, soft capsule, hard capsules, tablet, chewable tablet, quick-integrating tablet, syrup, liquid, suspension, suppository, ointment, cream, gel, adhesive, inhalant, injection and the like. A preparation thereof is prepared according to a conventional method. Since rare fatty acid derivatives are poorly soluble in water, they are dissolved in a non-hydrophilic organic solvent such as plant-derived oil, animal-derived oil and the like or dispersed or emulsified in an aqueous solution together with an emulsifier, a dispersing agent, a surfactant and the like by a homogenizer (high-pressure homogenizer) and used.

Examples of the additives that can be used for formulating include animal and plant oils such as soybean oil, safflower oil, olive oil, germ oil, sunflower oil, beef fat, sardine oil and the like, polyalcohols such as polyethylene glycol, propylene glycol, glycerol, sorbitol and the like, surfactants such as sorbitan ester of fatty acid, sucrose ester of fatty acid, glycerin fatty acid ester, polyglycerol ester of fatty acid and the like, excipients such as purified water, lactose, starch, crystalline cellulose, D-mannitol, lecithin, gum arabic, sorbitol solution, carbohydrate solution and the like, sweetener, colorant, pH adjuster, flavor and the like. A liquid preparation may be dissolved or suspended in water or other suitable medium when in use. Also, tablet and granules may be coated by a well-known method.

For administration in the form of an injection, intravenous, intraperitoneal, intramuscular, subcutaneous, transdermal, intraarticular, intrasynovial, intrathecal, intraperiosteum, sublingual, oral administrations and the like are preferable, and intravenous administration or intraperitoneal administration is particularly preferable. The intravenous administration may be any of drip administration and bolus administration.

When the anti-inflammatory agent for use according to the present invention is a food or a food additive, the form of the food is not particularly limited as long as it permits oral ingestion, such as solution, suspension, powder, solid formed article and the like. Specific examples include supplements (powder, granule, soft capsule, hard capsule, tablet, chewable tablet, quick-integrating tablet, syrup, liquid etc.), drinks (carbonic acid drinks, lactic acid drinks, sport drinks, fruit juice drinks, vegetable drinks, soymilk beverage, coffee drinks, tea drinks, powder drinks, concentrated drinks, nutrition drinks, alcohol drinks etc.), confectionery (gummy candy, jelly, gum, chocolate, cookie, candy, caramel, Japanese confectionery, snack etc.), instant food (instant noodles, retort food, can, microwavable foods, instant soup, miso soups, freeze-dried food etc.), oil, fats and oils food (mayonnaise, dressing, butter, cream, margarine etc.), wheat powder products (bread, pasta, noodle, cake mix, bread crumb etc.), seasoning (sauce, tomato processing seasoning, flavor seasoning, cooking mixture, soup etc.), processed meat products (meat ham, sausage etc.).

The above-mentioned foods can contain, where necessary, various nutrients, various vitamins (vitamin A, vitamin B1, vitamin B2, vitamin B6, vitamin C, vitamin D, vitamin E, vitamin K etc.), various minerals (magnesium, zinc, iron, sodium, potassium, selenium etc.), dietary fiber, dispersing agent, stabilizer such as emulsifier and the like, sweetener, flavor components (citric acid, malic acid etc.), flavor, royal jelly, propolis, Agaricus and the like.

When the anti-inflammatory agent for use according to the present invention is a feed or a feed additive, the feed is, for example, pet food, stock raising or aquaculture feed additive and the like.

When the anti-inflammatory agent for use according to the present invention is a cosmetic or a cosmetic additive, the cosmetic is, for example, cream, gel, skin milk, serum, toner, microemulsion essence, facial mask, foundation, lip rouge, eye shadow, shampoo, conditioner, bath additive and the like, and a flavor and the like may be mixed therewith.

Only one kind of rare fatty acid derivative may be blended with the pharmaceutical product, food, feed, cosmetic and the like for use according to the present invention or two or more kinds thereof may be used in combination.

The dose of the pharmaceutical product of the present invention or the ingestion amount of the food of the present invention can be appropriately determined according to the age and body weight of the patients or those who ingest same, symptom, administration time, dosage form, administration method, combination of medicaments and the like. For example, when the pharmaceutical product of the present invention is orally administered, the total amount of the rare fatty acid derivative as an active ingredient is 0.02 - 100 mg/kg body weight, preferably 0.2 - 50 mg/kg body weight, per day for an adult, or 0.002 mg - 50 mg/kg body weight, preferably 0.02 - 50 mg/kg body weight, by parenteral administration, which can be administered once a day or in several (2 - 5) portions per day. When it is ingested as a food, it can be added to a food such that the total ingestion amount of the rare fatty acid derivative as an active ingredient is 1 - 6000 mg, preferably 10 - 3000 mg, per day for an adult. The ingestion amount of the feed of the present invention and the amount of use of the cosmetic of the present invention can each appropriately determined according to the above-mentioned ingestion amount of the food and the above-mentioned dose of the pharmaceutical product.

The present invention is explained in more detail in the following by referring to Examples. The Examples are mere exemplifications of the present invention and do not limit the scope of the present invention in any manner.

### [Examples]

The following rare fatty acid derivatives (No. 4 - No. 23) used in the present invention were prepared based on the above-mentioned methods, and other fatty acids (No. 1 - No. 3, No. 24, LA, ALA, GLA) were purchased as general reagents. BAY11-7082, which is a IκBα phosphorylation inhibitor, was purchased from Cayman Chemical, and other reagents were purchased from Wako Pure Chemical Industries, Ltd. or Nacalai Tesque and others.
No. 1: trans-10,cis-12-octadecadienoic acid
No. 2: cis-9,trans-11-octadecadienoic acid
No. 3: trans-9,trans-11-octadecadienoic acid
No. 4: 10-hydroxy-cis-12-octadecenoic acid
No. 5: 10-hydroxy-octadecanoic acid
No. 6: 10-oxo-cis-12-octadecenoic acid
No. 7: 10-hydroxy-cis-12,cis-15-octadecadienoic acid
No. 8: 10-oxo-cis-12,cis-15-octadecadienoic acid
No. 9: 10-oxo-cis-6,cis-12-octadecadienoic acid
No. 10: 12-hydroxy-octadecanoic acid (Reference)
No. 11: 10-oxo-trans-11-octadecenoic acid (Reference)
No. 12: 10-oxo-trans-11,cis-15-octadecadienoic acid
No. 13: 10-oxo-cis-6,trans-11-octadecadienoic acid
No. 14: 13-hydroxy-cis-9-octadecenoic acid
No. 15: 13-oxo-cis-9-octadecenoic acid
No. 16: 13-oxo-cis-9,cis-15-octadecadienoic acid
No. 17: 13-oxo-cis-6,cis-9-octadecadienoic acid
No. 18: 10,13-dihydroxy-octadecanoic acid
No. 19: 13-hydroxy-cis-6,cis-9-octadecadienoic acid
No. 20: 10,13-dihydroxy-cis-6-octadecenoic acid
No. 21: 10-hydroxy-cis-6,cis-12-octadecadienoic acid
No. 22: 13-hydroxy-cis-9,cis-15-octadecadienoic acid
No. 23: 10,13-dihydroxy-cis-15-octadecenoic acid
No. 24: trans-9,trans-11,trans-13-octadecatrienoic acid
LA: linoleic acid
GLA: γ-linolenic acid
ALA: α-linolenic acid
tBHQ: tert-butylhydroquinone

### Example 1 (evaluation of NO production amount by Griess method)

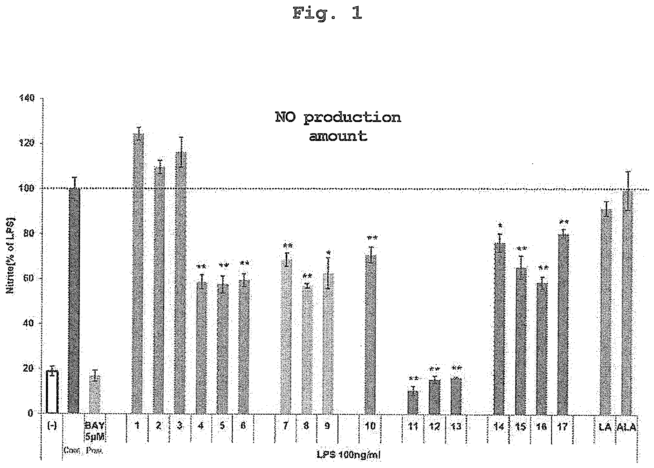

The mouse-derived macrophage-like cell line RAW264.7 was seeded in a 48 well plate 1×10⁵ cells per well. After culture for 5 hr, Escherichia coli-derived lipopolysaccharide (LPS) was added to 100 ng/mL over 24 hr to activate RAW264.7 cells. Simultaneously with LPS, various compounds (No. 1 - No. 17, LA, ALA) were added at 10 µM, and the concentration of nitrous acid (NO₂⁻), which is an oxide of nitric oxide (NO) produced by macrophage activation, in the culture medium was measured. The amount of NO₂ in the culture medium was quantified by the Griess method and in reference to Shan Lin et al., Molecular Nutrition and Food Research 57 (2013) p1135-1144, "Auraptene suppresses inflammatory responses in activated RAW264 macrophages by inhibiting p38 mitogen-activated protein kinase activation", the section of Material and methods Measurements of TNF-α, MCP-1, and NO concentrations. The quantification was actually performed as follows. RAW264.7 culture medium treated with LPS and various compounds for 24 hr was recovered. 0.2% N-(1-naphthyl) ethylenediamine and 2% Sulfanilamide/10% H₃PO₄ were blended at 1:1 immediately before reaction to give a Griess reagent, RAW264.7 culture medium and the Griess reagent were mixed at 1:1 and reacted at room temperature for 10 min. After completion of the reaction, absorbance at 550 nm was measured. NaNO₂ was used as the NO₂⁻ standard product, and the NO₂⁻ concentration in the culture medium was calculated. The concentration of the sample was adjusted with ethanol. Ethanol was used as a negative control, and BAY11-7082 (5 µM), which is a IκBα phosphorylation inhibitor, was used as a positive control. The results are shown in Fig. 1.

### Example 2 (evaluation of anti-stress activity to H₂O₂)

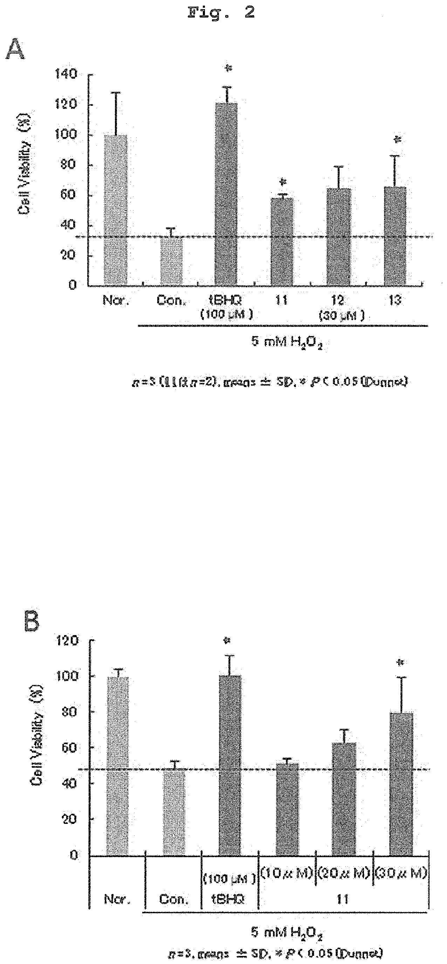

The cell line HepG2 derived from human liver cancer was cultured in D-MEM (10% FBS, 100 U/mL penicillin, 100 µg/mL streptomycin), seeded in a 96 well plate at 3.0x10⁴ cells/well, and cultured at 37°C, 5% CO₂ for 24 hr. Various compounds (tBHQ, No. 11 - No. 13) were dissolved in serum-free D-MEM (100 U/mL penicillin, 100 µg/mL streptomycin, 0.1% BSA, 0.3% ethanol) to prepare test media having adjusted concentrations. The supernatant was removed from the 96 well plate, the above-mentioned test medium was added at 100 µL/well, and the cells were cultured at 37°C, 5% CO₂ for 24 hr. Thereafter, the supernatant was removed again from the 96 well plate, 5 mM H₂O₂-containing D-MEM (100 U/mL penicillin, 100 µg/mL streptomycin) was added at 100 µL/well, 37°C, and the cells were cultured at 5% CO₂ for 30 min. The supernatant was removed from the 96 well plate, WST-1 reagent-containing D-MEM (7 µg/mL 1-Methosy PMS, 33 µg/mL WST-1) was added at 100 µL/well, the cells were cultured at 37°C, 5% CO₂ for 2 hr, and the absorbance at 450 nm was measured. The results are shown in Fig. 2A, B.

### Example 3 (effect on anti-oxidant enzyme HO-1 mRNA expression)

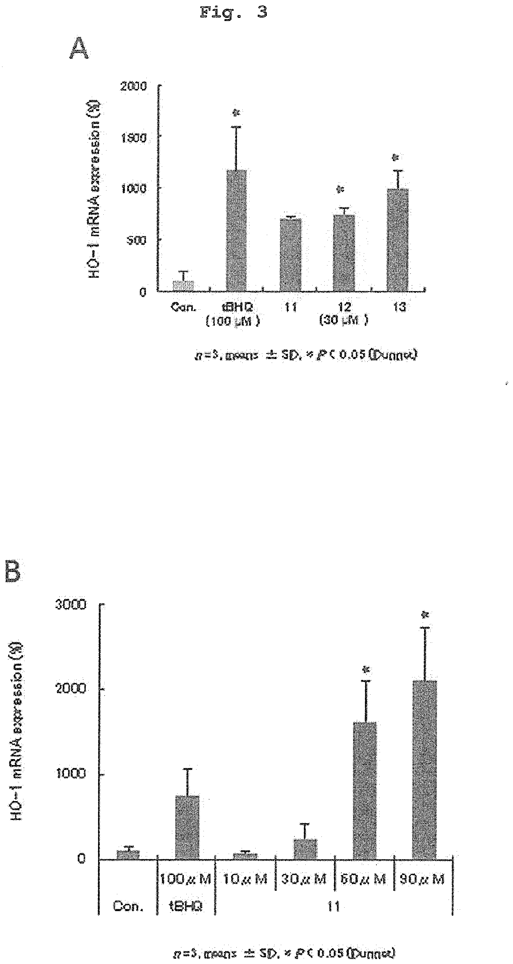

HepG2 cells were seeded in a 24 well plate at 1.5x10⁵ cells/well, and cultured at 37°C, 5% CO₂ for 24 hr. Various compounds (tBHQ, No. 11 - No. 13) were dissolved in serum-free D-MEM (100 U/mL penicillin, 100 µg/mL streptomycin, 0.1% BSA, 0.3% ethanol) to prepare test media having adjusted concentrations. The supernatant was removed from the 24 well plate, the above-mentioned test medium was added at 500 µL/well, and the cells were cultured at 37°C, 5% CO₂ for 6 hr. Thereafter, the supernatant was removed again from the 24 well plate, the cells were washed twice with sterile PBS, and the cells were recovered using 300 µL/well Sepasol. RNA was extracted from the recovered cells, and cDNA was synthesized. Real-time PCR method was performed using the cDNA as a template, and SYBR Green, and the expression of anti-oxidant enzyme HO-1 mRNA was evaluated. GAPDH was used as the internal standard, and 43 cycles of initial denaturation at 95°C for 15 min, amplification reaction at 95°C for 15 sec and 60°C for 30 sec were performed under PCR conditions. The results are shown in Fig. 3A, B.

### Example 4 (effect on intranuclear expression of transcription factor Nrf2 involved in anti-oxidation reaction)

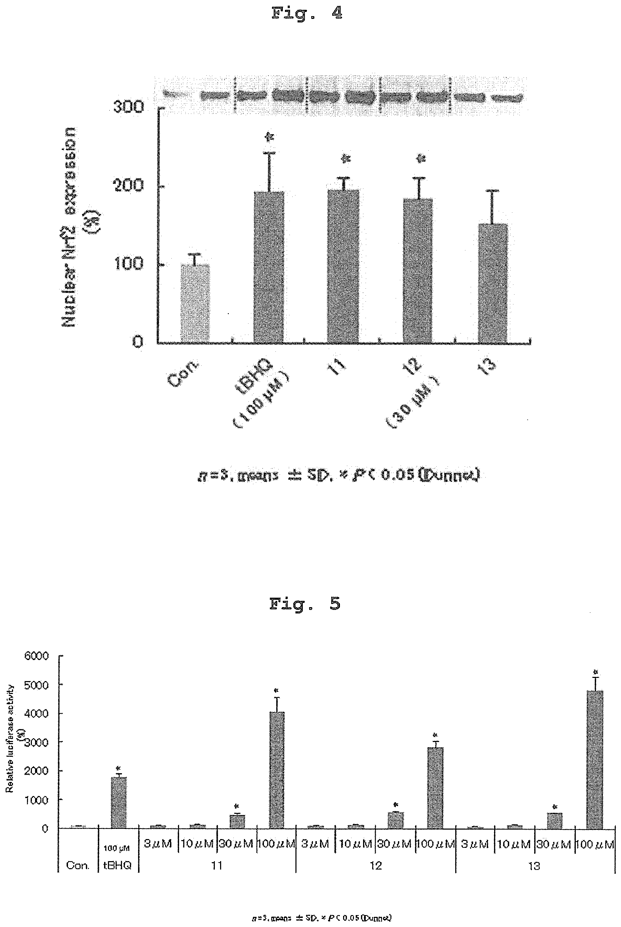

HepG2 cells were seeded in a 12 well plate at 3.0x10⁴ cells/well, and cultured at 37°C, 5% CO₂ for 24 hr. Various compounds (tBHQ, No. 11 - No. 13) were dissolved in serum-free D-MEM (100 U/mL penicillin, 100 µg/mL streptomycin, 0.1% BSA, 0.3% ethanol) to prepare test media having adjusted concentrations. The supernatant was removed from the 12 well plate, the above-mentioned test medium was added at 1 mL/well, and the cells were cultured at 37°C, 5% CO₂ for 24 hr. Thereafter, the supernatant was removed again from the 12 well plate, the cells were washed with sterile PBS, and dissolved in 100 µL of dissolution buffer (10 mM HEPES-KOH, 250 mM sucrose, 10 mM potassium chloride, 1.5 mM magnesium chloride, 1 mM EDTA, 1 mM EGTA, proteinase inhibitor cocktail, pH 7.6). The solution was centrifuged at 1,000xg, 4°C for 5 min, the supernatant was removed, cell pellets were dissolved in 30 µL of nucleus extraction buffer (20 mM HEPES-KOH, 100 mM sodium chloride, 1% SDS, 1 mM EDTA, 1 mM EGTA, proteinase inhibitor cocktail, pH 6.8), and the mixture was shaken at 4°C for 1 hr. Thereafter, the solution was centrifuged at 10,000×g, 4°C for 15 min, the supernatant was recovered to give a nucleoprotein fraction. The above-mentioned supernatant recovered as the nucleoprotein fraction was measured for the protein concentration by using Bio-Rad DC Protein Assay kit, diluted to the same protein amount and used as a sample. To the above-mentioned sample were added a loading buffer and 2-mercaptoethanol and the mixture was boiled at 95°C for 5 min. The above-mentioned boiled sample was applied by 20 µL each to 5% concentration gel and 10% electrophoresis gel, and the mixture was electrophoresed at 20 mA for 20 min and 150 V for 60 min. The protein was transferred from the gel on a PVDF membrane at 15 V for 35 min, the PVDF membrane after transfer was immersed in a blocking buffer, and stood overnight. Then, the PVDF membrane was reacted with a 400-fold diluted antihuman Nrf2 rabbit antibody for 2 hr, washed and reacted with 500-fold diluted HRP-labeled anti-rabbit IgG for 1 hr. Using Chemi-Lumi One Super, the band on the PVDF membrane was detected. The results are shown in Fig. 4.

### Example 5 (effect on transcription activity of transcription factor Nrf2 involved in anti-oxidation reaction)

Rat adrenal gland medulla pheochromocytoma PC12 cells incorporating a luciferase gene expression vector containing a luciferase gene linked to a rat NQO1 promoter region containing the binding region of rat Nrf2 were cultured in D-MEM (High-Glucose, 15% HS, 5% FBS, 300 mg/mL hygromycin B). The cells were seeded in a 96 well plate at PC12 cells 5.0×10⁴ cells/well, and cultured at 37°C, 5% CO₂ for 24 hr. Various compounds (tBHQ, No. 11 - No. 13) were dissolved in D-MEM (High- Glucose, 15% HS, 5% FBS, 300 mg/mL hygromycin B, 0.3% ethanol) to prepare test media having adjusted concentrations. The supernatant was removed from the 96 well plate, the above-mentioned test medium was added at 100 µL/well, and the cells were cultured at 37°C, 5% CO₂ for 9 hr. Thereafter, the supernatant was removed again from the 96 well plate, luminescence reagent was added at 50 µL/well, and the luminescence intensity was measured. The results are shown in Fig. 5.

### Example 6 (effect to differentiate macrophage into M2 macrophage)

C57BL/6N mouse (male, 6- to 12-week-old) (SHIMIZU experiment material) was sacrificed by cervical dislocation, and femur including the knee joint and the hip joint was isolated. The femur was transferred to a 100 mm dish (Thermo Fisher Scientific) containing RPMI 1640 medium (nacalai tesque), and the bone marrow fluid was collected on the dish from the femur. The cell aggregates in the dish were suspended, and placed in a 50 mL centrifugation tube. The suspended cell aggregates were centrifuged at 1500 rpm for 10 min, the supernatant was removed, the cell pellets were suspended in 2 mL of RBC lysis buffer (0.01 M Tris-HCl buffer containing 8.3 g/L ammonium chloride), and the suspension was stood for 3 min. Thereafter, the medium (8 mL) was added to quench the reaction by dilution, the mixture was centrifuged at 1500 rpm for 10 min, and the supernatant was removed. A single cell suspension of bone marrow-derived cells was prepared from the cell pellets by using a 40 µm-cell strainer (BD Bioscience).

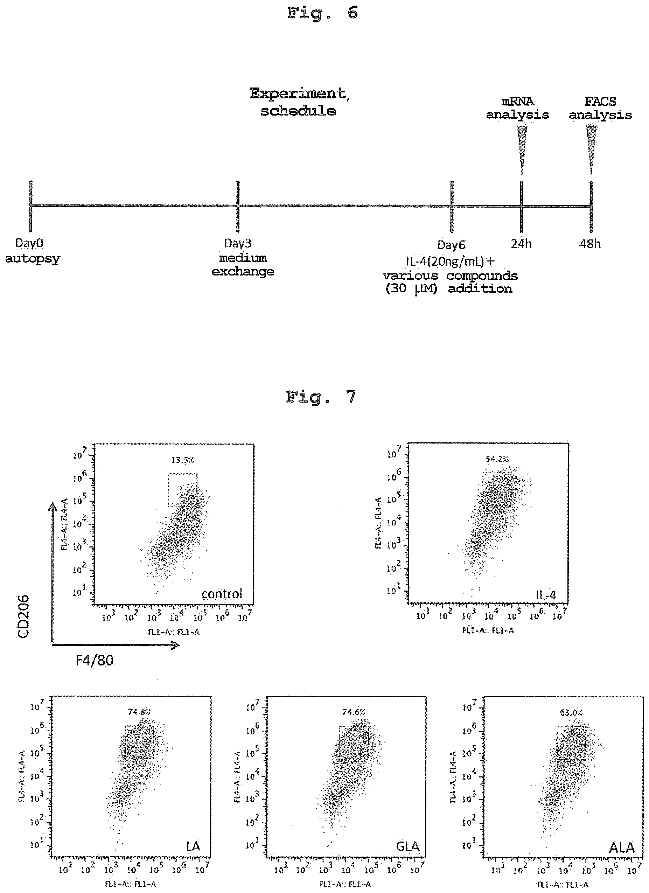

The viable cells in the above-mentioned bone marrow-derived cells were counted by using Trypan Blue staining solution (nacalai tesque), seeded in a 100 mm dish at a density of 1.2x10⁶ cells/dish, and cultured in a CO₂ incubator according to the schedule described in Fig. 6. As a cell medium, RPMI 1640 medium added with 10% FBS (Biowest), 1% Penicillin/Streptomycin (nacalai tesque), 20%(v/v) L929 conditioned medium and 55 µM β-mercaptoethanol (Gibco) was used. On day 6 of culture, IL-4 (20 ng/mL) and various compounds (LA, GLA, ALA, No.1-3, 7, 8, 9, 14-24) (30 µM) were added to the medium.

### (1) cell surface antigen expressing on M2 macrophage

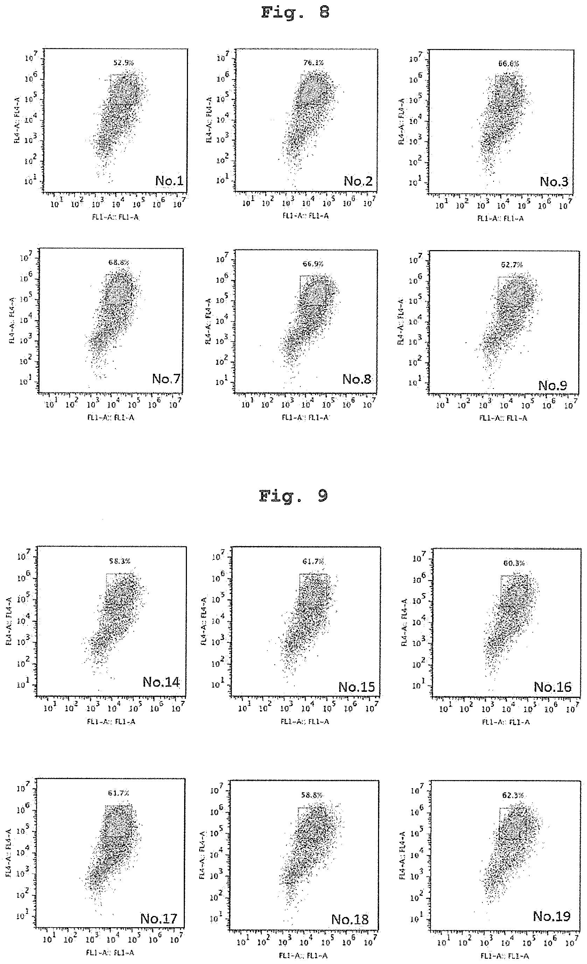

The cell surface antigen expressing on M2 macrophage at 48 hr after addition of IL-4 and various compounds (LA, GLA, ALA, No.1-3, 7, 8, 9, 14-24) was confirmed. First, the cells were recovered in a 4 mL Falcon tube (BD Bioscience), and centrifuged at 1500 rpm for 5 min to give cell pellets. The cells were washed with FACS buffer (0.1% BSA (fatty acid free) (nacalai tesque), and the cell pellets were uniformized by tapping. Furthermore, BD pharmingen™ Purified Rat anti-Mouse CD16/CD32 (BD Bioscience) was added, and the mixture was tapped and stood at 4°C for 10 min (Fc blocking). To detect the cell surface antigen (CD206, F4/80) expressing on M2 macrophage, a fluorochrome-labeled monoclonal antibody (Anti-Mouse CD206 Alexa Fluor 647 (BioLegend), Anti-Mouse F4/80 Antigen FITC (eBioscience)) was added, and the mixture was tapped and stood at 4°C for 30 min. Thereafter, the cells were washed with FACS buffer, the cell pellets were uniformized by tapping, and Viability Dye 7-AAD (eBioscience) was added. The cell surface antigen was measured by BD Accuri™ C6 flow cytometer (BD Bioscience). For the analysis, FlowJo™ (Tomy Digital Biology) was used. The results are shown in Figs. 7 - 10.

### (2) mRNA expression of M2 macrophage

At 24 hr after addition of IL-4 and various compounds (ALA, No.7, 8, 16, 22, 23), mRNA (Arginase 1, IL-1β) expression of the cells was confirmed. First, cells were seeded in a well, 700 µL of Sepasol RNAIsuper (nacalai tesque) was added and the mixture was shaken for 30 min. The cells were dispersed and the dispersion was transferred into a 1.5 mL tube. To the 1.5 mL tube was added 150 µL of chloroform (nacalai tesque), mixed by inverting, stood at room temperature for 5 min, and centrifuged at 4°C, 15,000 rpm for 65 min. The aqueous layer (350 µL) was transferred into another 1.5 mL tube, 350 µL of isopropanol (nacalai tesque) were further added. The tube was mixed by inverting, stood at room temperature for 15 min, and centrifuged at 4°C, 15,000 rpm for 65 min, and the supernatant was removed. To the precipitate was added 500 µL of 75% ethanol (nacalai tesque) and the mixture was centrifuged at 4°C, 15,000 rpm for 15 min, and the upper layer was removed. This operation was performed twice in total. The obtained precipitate (Total RNA) was air dried for about 40 min, dissolved in 20 µL of Ultra pure water (Invitrogen), and the mRNA concentration was measured by Nano Drop (Scrum). The mRNA concentration was adjusted with Ultra pure water to 1000 ng/µL on ice to give an RNA solution. Oligo dT primer (Gibco) (1 µL) and RNA solution (10 µL) (1000 ng/µL) were added into a 0.2 mL 8-tube, incubated in a Thermal Cycler at 70°C for 10 min to destroy the higher order structure of RNA. Furthermore, the reagents shown in Table 1 were added to the 0.2 mL 8-tube, and the mixture was incubated in a Thermal Cycler (TAKARA) at 42°C for 50 min and at 70°C for 15 min. After cooling on ice, the reaction mixture was gathered on the bottom of the tube by light centrifugation, and cDNA was obtained.

**Table 1**

| | | |
|---|---|---|
| RNA sample/primer mixture | 10 | µl |
| 5 x reverse transcription buffer (Promega) | 4 | µl |
| RNase inhibiter (TOYOBO) | 0.5 | µl |
| 2.5 mM dNTP Mix (TAKARA) | 2 | µl |
| Nuclease Free Water | 2.5 | µl |
| Superscript (II) reverse transcriptase (Promega) | 1 | µl |
| Total | 20 | µl |

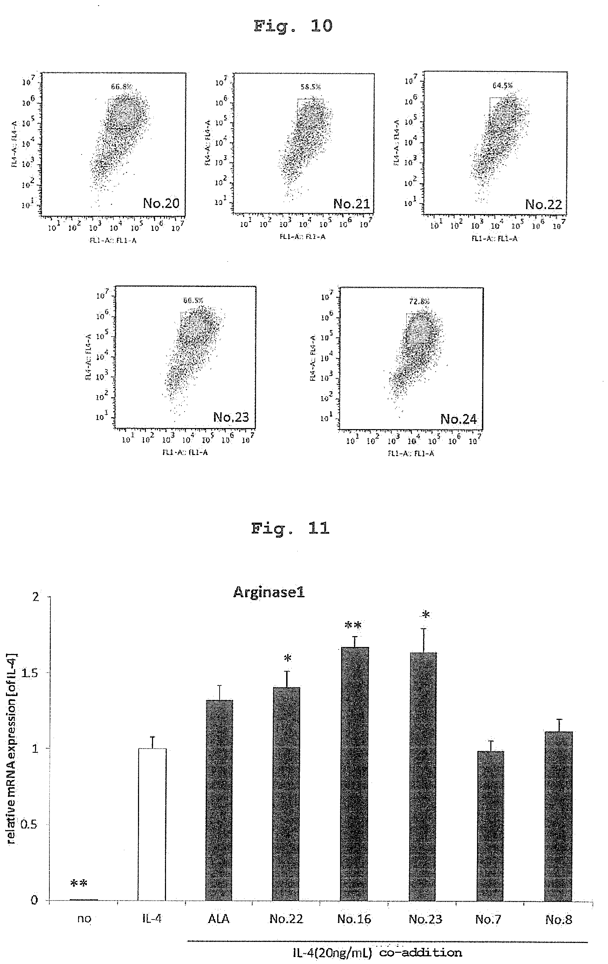

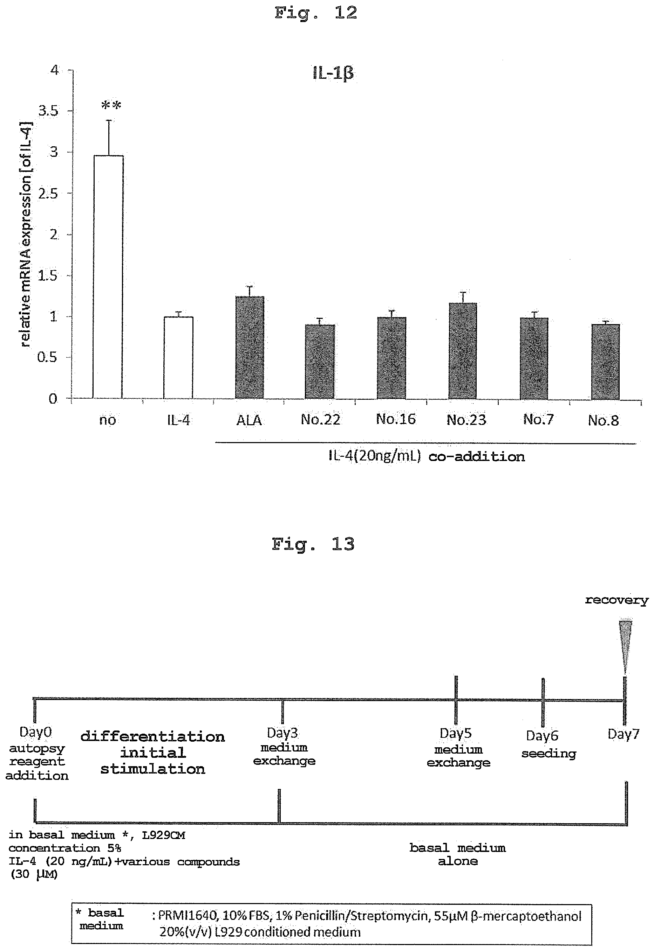

The obtained cDNA was diluted 5-fold with Ultra pure water. A plasmid solution (5 µL) to be used as the standard was taken in a 0.6 mL tube, and diluted 10-fold with Ultra pure water (45 µL) . This operation was repeated, and a plasmid solution diluted 10²- to 10⁹-fold was produced. The reagent shown in Table 2 per sample was prepared in a 1.5 mL tube and dispensed to a 96 well plate by 12 µL each. Ultra pure water as a negative control, the dilution solution (10³-10⁸ dilution) at each concentration produced above as the standard, and cDNA of a sample for measurement were added by 3 µL each to the plate. The plate was set in Light-Cycler™ (Roche), and PCR was performed. With each of the Arginase 1 mRNA expression level and IL-1β expression level of the cells added with IL-4 alone as 1, the Arginase 1 mRNA expression level and IL-1β expression level when IL-4 and various compounds were concurrently added are shown in Figs. 11, 12.

**Table 2**

| | | |
|---|---|---|
| dH₂O | 8.4 | µl |
| SYBR Green (TOYOBO) | 8.0 | µl |
| Total | 0.8 | µl |

### Example 7 (effect of differentiation of monocyte into M2 macrophage)

It is considered that, in the body, an inflammatory macrophage is released in blood in a monocyte state and, after circulation in the whole body, infiltrates into the peripheral tissues, and differentiates into a tissue-specific macrophage according to the environment of the peripheral tissues. In this Example, whether addition of various compounds of fatty acid derivative in the initial stage of differentiation of monocyte into macrophage promotes induction of differentiation of monocyte into M2 macrophage was studied.

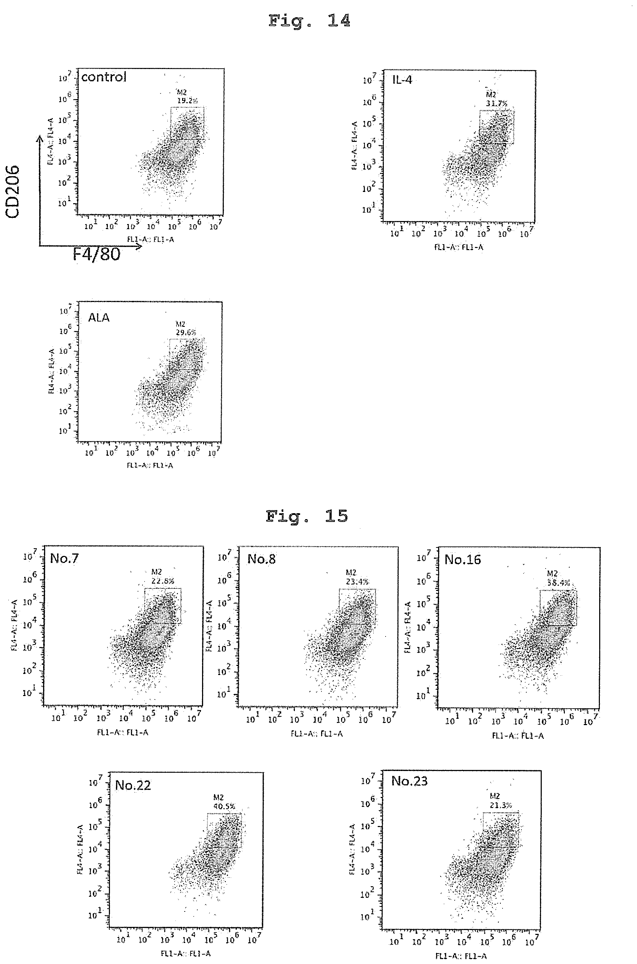

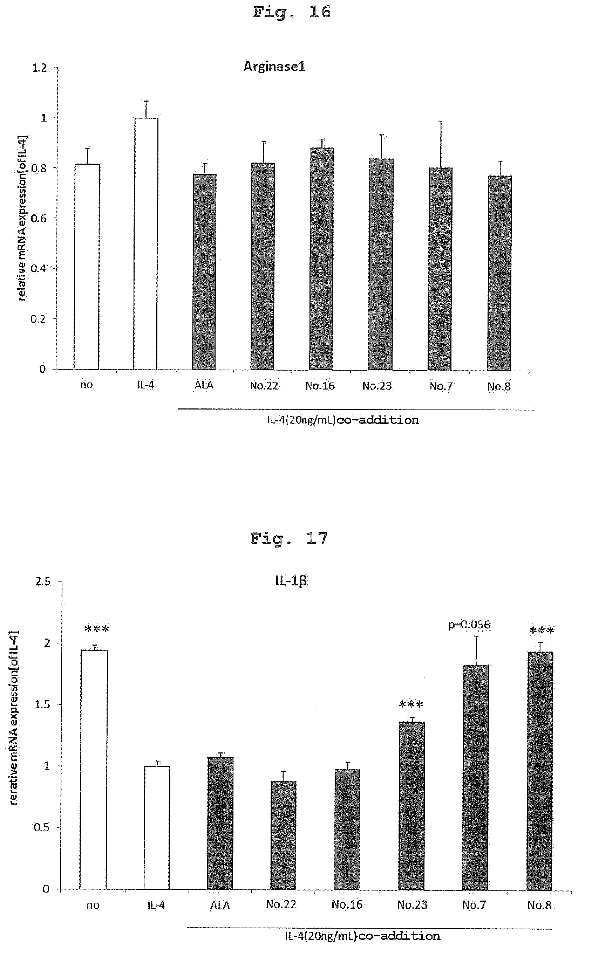

Using the bone marrow-derived cells prepared in Example 6 and considering from the initial day of culture to day 3 of culture as the initial stage of differentiation, culture according to the schedule described in Fig. 13 was performed. As the cell medium, RPMI 1640 medium added with 10% FBS, 1% Penicillin/Streptomycin, 5%(v/v) L929 conditioned medium and 55 µM β-mercaptoethanol (Gibco), IL-4 (20 ng/mL) and various compounds (ALA, No.7, 8, 16, 22, 23) (30 µM) was used from the initial day of culture to day 3 of culture. After day 3 of culture, RPMI 1640 medium added with 10% FBS, 1% Penicillin/Streptomycin, 20%(v/v) L929 conditioned medium and 55 µM β-mercaptoethanol (Gibco) was used. The cells on day 7 of culture were recovered, and mRNA expression and cell surface antigen were confirmed in the same manner as in Example 6. The results are shown in Figs. 14 - 17.

### Example 8 (effect on intestinal mucosa immune system to differentiate monocyte into M2 macrophage)

It is known that various environmental factors such as cytokine and the like in the peripheral tissue are important for the differentiation of monocyte into M2 macrophage. Therefore, whether a Th2 cytokine dominant environment can be formed in the intestine as a result of an interaction between immunocyte and various compounds of fatty acid derivatives was studied.

BALB/c mouse (♂, 6- to 12-week-old) (fresh water experiment material) was sacrificed by cervical dislocation, and the mesenteric lymph node and Peyer's patch were isolated. Each tissue was finely cut, and treated with RPMI 1640 (5% FBS, 1.0 mg/mL Collagenase containing) medium at 37°C for 30 min. The tissue was further mashed with the plunger part of a 1 mL syringe to give a cell suspension. The cell suspension was centrifuged at 1500 rpm for 10 min, the supernatant was removed and, using a 40 µm-cell strainer, a single cell suspension of the mesentericlymph node-derived cells and a single cell suspension of the Peyer's patch-derived cells were prepared.

In addition, BALB/c mouse was sacrificed by cervical dislocation, and the spleen was isolated. The spleen was mashed on a 100 mm dish to give a cell suspension. The cell aggregates in the dish were suspended, and placed in a 50 mL centrifugation tube. The suspended cell aggregates were centrifuged at 1500 rpm for 10 min, the supernatant was removed, the cell pellets were suspended in 2 mL of RBC lysis buffer, and the suspension was stood for 3 min. Thereafter, the medium (8 mL) was added to quench the reaction by dilution, the mixture was centrifuged at 1500 rpm for 10 min, and the supernatant was removed. A single cell suspension of spleen-derived cells was prepared from the cell pellets by using a 40 µm-cell strainer.

The viable cells in each of the above-mentioned mesentericlymph node-derived cells, Peyer's patch-derived cells and spleen-derived cells were counted by using Trypan Blue staining solution, seeded at 1.0x10⁶ cells/well in a 96 well plate, various compounds (ALA, No.7, 8, 16, 22, 23) were added and the cells were cultured for 18 - 24 hr in the presence of lymphocyte activators PMA and Ionomycin. After culture, each supernatant was recovered. As the medium for culture, RPMI 1640 added with 10% FBS, 1% Penicillin/Streptomycin was used.

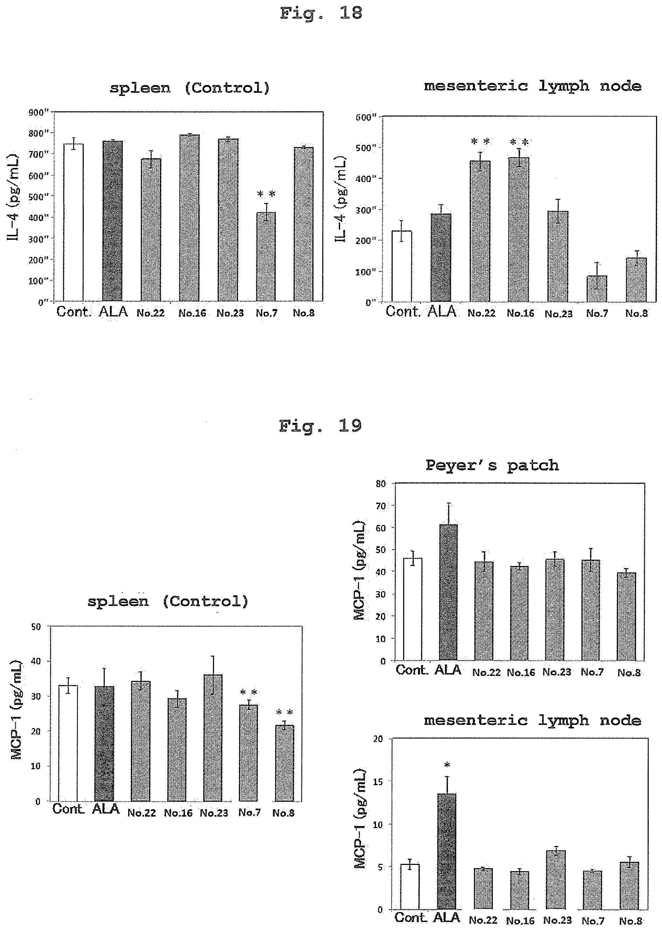

The concentration of IL-4 and MCP-1 contained in the each recovered supernatant was measured by ELISA. Basically, the recommended protocol of ELISA Ready-SET-Go!^{(R)} (eBioscience) was adopted. Capture antibody was diluted with a coating buffer, added to a 96 well plate at 50 µL/well, and the plate was sealed and stood overnight at 4°C. The well was washed 3 times with 250 µL/well of Wash buffer, ELISA/ELISPOT Diluent was added to the well at 100 µL/well, and the mixture was stood at room temperature for 1 hr. 50 µL/well of standard and each supernatant were added to the well, the well was stood at room temperature for 2 - 3 hr and washed 3 times with Wash buffer at 250 µL/well. Detection antibody was diluted with ELISA/ELISPOT Diluent, added to the well at 50 µL/well, the well was stood at room temperature for 1 hr, and washed 3 times with 250 µL/well of Wash buffer. Avidin-HRP was diluted with ELISA/ELISPOT Diluent, was added to the well at 50 µL/well, and the well was stood at room temperature for 15 min and washed 6 times with 250 µL/well of Wash buffer. TMB solution was added to the well at 50 µL/well, and the well was stood at room temperature for 15 min. 1 M H₃PO₄ solution was added to the well at 25 µL/well to discontinue the enzyme reaction. The absorbance at wavelength 450 nm was measured by a Microplate Reader. The results are shown in Figs. 18, 19.

While the present invention has been described with emphasis on preferred embodiments, it is obvious to those skilled in the art that the preferred embodiments can be modified.

### [Industrial Applicability]

The present invention has clarified that rare fatty acid derivatives have a conventionally-unknown anti-inflammatory effect as a physiological function thereof. An anti-inflammatory agent containing the rare fatty acid derivative is applicable to various fields such as pharmaceutical product, food, feed and the like, and the present invention is industrially extremely useful.

This application is based on patent application Nos. 2014-011866 (filing date: January 24, 2014) and 2014-162982 (filing date: August 8, 2014) filed in Japan.

## Claims

1. An agent comprising the following fatty acid:
(1) a saturated fatty acid or an unsaturated fatty acid having a trans double bond at the 11-position or at least one cis double bond at the 6-position, the 12-position, the 15-position, which has 18 carbon atoms and a hydroxyl group or carbonyl group at the 10-position selected from the group consisting of 10-hydroxy-cis-12-octadecenoic acid, 10-hydroxy-cis-12,cis-15-octadecadienoic acid, 10-hydroxy-cis-6,cis-12-octadecadienoic acid, 10-hydroxy-cis-6,cis-12,cis-15-octadecatrienoic acid, 10,12-dihydroxy-octadecanoic acid, 10-hydroxy-octadecanoic acid, 10-hydroxy-cis-15-octadecenoic acid, 10-hydroxy-cis-6-octadecenoic acid, 10-hydroxy-cis-6,cis-15-octadecadienoic acid, 10-hydroxy-trans-11-octadecenoic acid, 10-hydroxy-trans-11,cis-15-octadecadienoic acid, 10-hydroxy-cis-6,trans-11-octadecadienoic acid, 10-hydroxy-cis-6,trans-11,cis-15-octadecatrienoic acid, 10-oxo-cis-12-octadecenoic acid, 10-oxo-cis-12,cis-15-octadecadienoic acid, 10-oxo-cis-6,cis-12-octadecadienoic acid, 10-oxo-cis-6,cis-12,cis-15-octadecatrienoic acid, 10-oxo-octadecanoic acid, 10-oxo-cis-6-octadecenoic acid, 10-oxo-cis-15-octadecenoic acid, 10-oxo-cis-6,cis-15-octadecadienoic acid, 10-oxo-cis-6,trans-11-octadecadienoic acid, 10-oxo-trans-11,cis-15-octadecadienoic acid, and 10-oxo-cis-6,trans-11,cis-15-octadecatrienoic acid,
(2) a saturated fatty acid or an unsaturated fatty acid having at least one cis double bond at the 6-position, the 9-position, the 15-position, which has 18 carbon atoms and a hydroxyl group or carbonyl group at the 13-position selected from the group consisting of 13-hydroxy-cis-9-octadecenoic acid, 13-hydroxy-cis-6,cis-9-octadecadienoic acid, 13-hydroxy-cis-9,cis-15-octadecadienoic acid, 13-hydroxy-cis-6,cis-9,cis-15-octadecatrienoic acid, 10,13-dihydroxy-octadecanoic acid, 10,13-dihydroxy-cis-6-octadecenoic acid, 10,13-dihydroxy-cis-15-octadecenoic acid, 10,13-dihydroxy-cis-6,cis-15-octadecadienoic acid, 10-oxo-13-hydroxy-cis-6-octadecenoic acid, 10-oxo-13-hydroxy-cis-15-octadecenoic acid, 10-oxo-13-hydroxy-cis-6,cis-15-octadecadienoic acid, 13-hydroxy-cis-5,cis-9-octadecadienoic acid, 13-hydroxy-trans-5,cis-9-octadecadienoic acid, 13-oxo-cis-9-octadecenoic acid, 13-oxo-cis-6,cis-9-octadecadienoic acid, 13-oxo-cis-9,cis-15-octadecadienoic acid, 13-oxo-cis-6,cis-9,cis-15-octadecatrienoic acid, 10,13-dioxo-octadecanoic acid, 10,13-dioxo-cis-6-octadecenoic acid, 10,13-dioxo-cis-15-octadecenoic acid, 10,13-dioxo-cis-6,cis-15-octadecadienoic acid, 13-oxo-cis-5,cis-9-octadecadienoic acid, and 13-oxo-trans-5,cis-9-octadecadienoic acid,
(3) a saturated fatty acid or an unsaturated fatty acid having at least one cis double bond at the 5-position, the 8-position, the 9-position, the 14-position, the 17-position, which has 18 or 20 carbon atoms and a hydroxyl group or carbonyl group at the 12-position selected from the group consisting of 12-hydroxy-cis-14-eicosenoic acid, 12-hydroxy-cis-14,cis-17-eicosadienoic acid, 12-hydroxy-cis-8,cis-14-eicosadienoic acid, 12-hydroxy-cis-5,cis-8-eicosadienoic acid, 12-hydroxy-cis-8,cis-14,cis-17-eicosatrienoic acid, 12-hydroxy-cis-5,cis-8,cis-14-eicosatrienoic acid, 12-oxo-octadecanoic acid, 12-oxo-cis-14-eicosenoic acid, 12-oxo-cis-14,cis-17-eicosadienoic acid, 12-oxo-cis-8,cis-14-eicosadienoic acid, 12-oxo-cis-5,cis-8-eicosadienoic acid, 12-oxo-cis-8,cis-14,cis-17-eicosatrienoic acid, and 12-oxo-cis-5,cis-8,cis-14-eicosatrienoic acid,
(4) an unsaturated fatty acid having at least one cis double bond at the 5-position, the 8-position, the 11-position, the 17-position, which has 20 carbon atoms and a hydroxyl group or carbonyl group at the 15-position selected from the group consisting of 15-hydroxy-cis-11-eicosenoic acid, 15-hydroxy-cis-11,cis-17-eicosadienoic acid, 15-hydroxy-cis-8,cis-11-eicosadienoic acid, 15-hydroxy-cis-5,cis-8,cis-11-eicosatrienoic acid, 15-hydroxy-cis-8,cis-11,cis-17-eicosatrienoic acid, 15-hydroxy-cis-5,cis-11-eicosadienoic acid, 15-hydroxy-cis-5,cis-11,cis-17-eicosatrienoic acid, 15-oxo-cis-11-eicosenoic acid, 15-oxo-cis-11,cis-17-eicosadienoic acid, 15-oxo-cis-8,cis-11-eicosadienoic acid, 15-oxo-cis-5,cis-8,cis-11-eicosatrienoic acid, 15-oxo-cis-8,cis-11,cis-17-eicosatrienoic acid, 15-oxo-cis-5,cis-11-eicosadienoic acid, and 15-oxo-cis-5,cis-11,cis-17-eicosatrienoic acid, or
(5) a saturated fatty acid having 16 carbon atoms and a hydroxyl group or carbonyl group at the 10-position selected from the group consisting of 10-hydroxy-hexadecanoic acid and 10-oxo-hexadecanoic acid,
for use for the prophylaxis or treatment of an inflammatory disease.

2. The agent for use according to claim 1, comprising the following fatty acid:
(1) a saturated fatty acid or an unsaturated fatty acid having a trans double bond at the 11-position or at least one cis double bond at the 6-position, the 12-position, which has 18 carbon atoms and a hydroxyl group or carbonyl group at the 10-position selected from the group consisting of 10-hydroxy-cis-12-octadecenoic acid, 10-hydroxy-octadecanoic acid, 10-oxo-cis-12-octadecenoic acid, 10-hydroxy-cis-12,cis-15-octadecadienoic acid, 10-hydroxy-cis-6,cis-12-octadecadienoic acid, 10-oxo-cis-12,cis-15-octadecadienoic acid, 10-oxo-cis-6,cis-12-octadecadienoic acid, 10-oxo-trans-11,cis-15-octadecadienoic acid and 10-oxo-cis-6,trans-11-octadecadienoic acid, or
(2) a saturated fatty acid or an unsaturated fatty acid having at least one cis double bond at the 6-position, the 9-position, the 15-position, which has 18 carbon atoms and a hydroxyl group or carbonyl group at the 13-position selected from the group consisting of 13-hydroxy-cis-9-octadecenoic acid, 13-hydroxy-cis-6,cis-9-octadecadienoic acid, 13-hydroxy-cis-9,cis-15-octadecadienoic acid, 10,13-dihydroxy-octadecanoic acid, 10,13-dihydroxy-cis-6-octadecenoic acid, 10,13-dihydroxy-cis-15-octadecenoic acid, 13-oxo-cis-9-octadecenoic acid, 13-oxo-cis-6,cis-9-octadecadienoic acid, and 13-oxo-cis-9,cis-15-octadecadienoic acid.

3. The agent for use according to claim 1 or 2 for use for the prophylaxis or improvement of an inflammatory disease involving macrophage.

4. The agent for use according to claim 1 or 2, wherein said agent is a food or a food additive.

5. The agent for use according to claim 1 or 2, wherein said agent is a pharmaceutical product.

6. The agent for use according to claim 1 or 2, wherein said agent is a feed or a feed additive.

## Patentansprüche

1. Mittel, das die folgende Fettsäure umfasst:
(1) eine gesättigte Fettsäure oder eine ungesättigte Fettsäure, die eine trans-Doppelbindung auf der 11-Position oder wenigstens eine cis-Doppelbindung auf der 6-Position, der 12-Position, der 15-Position aufweist, welche 18 Kohlenstoffatome und eine Hydroxygruppe aufweist, oder eine Carbonylgruppe auf der 10-Position aufweist und die aus der Gruppe ausgewählt ist, die aus 10-Hydroxy-cis-12-octadecensäure, 10-Hydroxy-cis-12,cis-15-octadecadiensäure, 10-Hydroxy-cis-6,cis-12-octadecadien-säure, 10-Hydroxy-cis-6,cis-12,cis-15-octadecatriensäure, 10,12-Dihydroxyoctadecansäure, 10-Hydroxyoctadecansäure, 10-Hydroxy-cis-15-octadecensäure, 10-Hydroxy-cis-6-octadecensäure, 10-Hydroxy-cis-6,cis-15-octadecadiensäure, 10-Hydroxy-trans-11-octadecensäure, 10-Hydroxy-trans-11,cis-15-octadecadiensäure, 10-Hydroxy-cis-6,trans-11-octadeca-diensäure, 10-Hydroxy-cis-6,trans-11,cis-15-octadecatriensäure, 10-Oxo-cis-12-octadecensäure, 10-Oxo-cis-12,cis-15-octadecadiensäure, 10-Oxo-cis-6,cis-12-octadecadiensäure, 10-Oxo-cis-6,cis-12,cis-15-octadecatrien-säure, 10-Oxooctadecansäure, 10-Oxo-cis-6-octadecensäure, 10-Oxo-cis-15-octadecensäure, 10-Oxo-cis-6,cis-15-octadecadiensäure, 10-Oxo-cis-6,trans-11-octadecadiensäure, 10-Oxo-trans-11,cis-15-octadecadiensäure und 10-Oxo-cis-6,trans-11,cis-15-octadecatriensäure besteht;
(2) eine gesättigte Fettsäure oder eine ungesättigte Fettsäure, die wenigstens eine cis-Doppelbindung auf der 6-Position, der 9-Position, der 15-Position aufweist, welche 18 Kohlenstoffatome und eine Hydroxygruppe aufweist, oder eine Carbonylgruppe auf der 13-Position aufweist und die aus der Gruppe ausgewählt ist, die aus 13-Hydroxy-cis-9-octadecensäure, 13-Hydroxy-cis-6,cis-9-octadecadiensäure, 13-Hydroxy-cis-9,cis-15-octadecadiensäure, 13-Hydroxy-cis-6,cis-9,cis-15-octadecatriensäure, 10,13-Dihydroxyoctadecansäure, 10,13-Dihydroxy-cis-6-octadecensäure, 10,13-Dihydroxy-cis-15-octadecensäure, 10,13-Dihydroxy-cis-6,cis-15-octadecadiensäure, 10-Oxo-13-hydroxy-cis-6-octadecensäure, 10-Oxo-13-hydroxy-cis-15-octadecensäure, 10-Oxo-13-hydroxy-cis-6,cis-15-octadecadiensäure, 13-Hydroxy-cis-5,cis-9-octadecadiensäure, 13-Hydroxy-trans-5,cis-9-octadecadiensäure, 13-Oxo-cis-9-octadecensäure, 13-Oxo-cis-6,cis-9-octadecadiensäure, 13-Oxo-cis-9,cis-15-octadecadiensäure, 13-Oxo-cis-6,cis-9,cis-15-octadecatriensäure, 10,13-Dioxooctadecansäure, 10,13-Dioxo-cis-6-octadecensäure, 10,13-Dioxo-cis-15-octadecensäure, 10,13-Dioxo-cis-6,cis-15-octadecadiensäure, 13-Oxo-cis-5,cis-9-octadecadiensäure und 13-Oxo-trans-5,cis-9-octadecadiensäure besteht;
(3) eine gesättigte Fettsäure oder eine ungesättigte Fettsäure, die wenigstens eine cis-Doppelbindung auf der 5-Position, der 8-Position, der 9-Position, der 14-Position, der 17-Position aufweist, welche 18 oder 20 Kohlenstoffatome und eine Hydroxygruppe aufweist, oder eine Carbonylgruppe auf der 12-Position aufweist und die aus der Gruppe ausgewählt ist, die aus 12-Hydroxy-cis-14-eicosensäure, 12-Hydroxy-cis-14,cis-17-eicosadiensäure, 12-Hydroxy-cis-8,cis-14-eicosadiensäure, 12-Hydroxy-cis-5,cis-8-eicosadiensäure, 12-Hydroxy-cis-8,cis-14,cis-17-eicosatriensäure, 12-Hydroxy-cis-5,cis-8,cis-14-eicosatriensäure, 12-Oxo-octadecansäure, 12-Oxo-cis-14-eicosensäure, 12-Oxo-cis-14,cis-17-eicosadiensäure, 12-Oxo-cis-8,cis-14-eicosadiensäure, 12-Oxo-cis-5,cis-8-eicosadiensäure, 12-Oxo-cis-8,cis-14,cis-17-eicosatriensäure und 12-Oxo-cis-5,cis-8,cis-14-eicosatriensäure besteht;
(4) eine ungesättigte Fettsäure, die wenigstens eine cis-Doppelbindung auf der 5-Position, der 8-Position, der 11-Position, der 17-Position aufweist, welche 20 Kohlenstoffatome und eine Hydroxygruppe aufweist, oder eine Carbonylgruppe auf der 15-Position aufweist und die aus der Gruppe ausgewählt ist, die aus 15-Hydroxy-cis-11-eicosensäure, 15-Hydroxy-cis-11,cis-17-eicosadiensäure, 15-Hydroxy-cis-8,cis-11-eicosadiensäure, 15-Hydroxy-cis-5,cis-8,cis-11-eicosatriensäure, 15-Hydroxy-cis-8,cis-11,cis-17-eicosatriensäure, 15-Hydroxy-cis-5,cis-11-eicosadiensäure, 15-Hydroxy-cis-5,cis-11,cis-17-eicosatriensäure, 15-Oxo-cis-11-eicosensäure, 15-Oxo-cis-11,cis-17-eicosadiensäure, 15-Oxo-cis-8,cis-11-eicosadiensäure, 15-Oxo-cis-5,cis-8,cis-11-eicosatriensäure, 15-Oxo-cis-8,cis-11,cis-17-eicosatriensäure, 15-Oxo-cis-5,cis-11-eicosadiensäure und 15-Oxo-cis-5,cis-11,cis-17-eicosatriensäure besteht; oder
(5) eine gesättigte Fettsäure, die 16 Kohlenstoffatome und eine Hydroxygruppe oder eine Carbonylgruppe auf der 10-Position aufweist und die aus der Gruppe ausgewählt ist, die aus 10-Hydroxyhexadecansäure und 10-Oxohexadecansäure besteht;
zur Verwendung für die Prophylaxe oder Behandlung einer Entzündungserkrankung.

2. Mittel zur Verwendung gemäß Anspruch 1, das die folgende Fettsäure umfasst:
(1) eine gesättigte Fettsäure oder eine ungesättigte Fettsäure, die eine trans-Doppelbindung auf der 11-Position oder wenigstens eine cis-Doppelbindung auf der 6-Position, der 12-Position aufweist, welche 18 Kohlenstoffatome und eine Hydroxygruppe aufweist, oder eine Carbonylgruppe auf der 10-Position aufweist und die aus der Gruppe ausgewählt ist, die aus 10-Hydroxy-cis-12-octadecensäure, 10-Hydroxyoctadecansäure, 10-Oxo-cis-12-octadecensäure, 10-Hydroxy-cis-12,cis-15-octadecadiensäure, 10-Hydroxy-cis-6,cis-12-octadecadiensäure, 10-Oxo-cis-12,cis-15-octadecadiensäure, 10-Oxo-cis-6,cis-12-octadecadiensäure, 10-Oxotrans-11,cis-15-octadecadiensäure und 10-Oxo-cis-6,trans-11-octadecadiensäure besteht; oder
(2) eine gesättigte Fettsäure oder eine ungesättigte Fettsäure, die wenigstens eine cis-Doppelbindung auf der 6-Position, der 9-Position, der 15-Position aufweist, welche 18 Kohlenstoffatome und eine Hydroxygruppe aufweist, oder eine Carbonylgruppe auf der 13-Position aufweist und die aus der Gruppe ausgewählt ist, die aus 13-Hydroxy-cis-9-octadecensäure, 13-Hydroxy-cis-6,cis-9-octadecadiensäure, 13-Hydroxy-cis-9,cis-15-octadecadiensäure, 10,13-Dihydroxyoctadecansäure, 10,13-Dihydroxy-cis-6-octadecensäure, 10,13-Dihydroxy-cis-15-octadecensäure, 13-Oxo-cis-9-octadecensäure, 13-Oxo-cis-6,cis-9-octadecadiensäure und 13-Oxo-cis-9,cis-15-octadecadiensäure besteht.

3. Mittel zur Verwendung gemäß Anspruch 1 oder 2 zur Verwendung für die Prophylaxe oder Linderung einer Entzündungserkrankung, bei der Makrophagen beteiligt sind.

4. Mittel zur Verwendung gemäß Anspruch 1 oder 2, wobei das Mittel ein Lebensmittel oder Lebensmittelzusatzstoff ist.

5. Mittel zur Verwendung gemäß Anspruch 1 oder 2, wobei das Mittel ein pharmazeutisches Produkt ist.

6. Mittel zur Verwendung gemäß Anspruch 1 oder 2, wobei das Mittel ein Futtermittel oder Futtermittelzusatzstoff ist.

## Revendications

1. Agent comprenant l'acide gras suivant :
(1) un acide gras saturé ou un acide gras insaturé ayant une double liaison trans en position 11 ou au moins une double liaison cis en position 6, en position 12, en position 15, qui a 18 atomes de carbone et un groupe hydroxyle ou un groupe carbonyle en position 10, choisi dans l'ensemble constitué par l'acide 10-hydroxy-cis-12-octadécénoïque, l'acide 10-hydroxy-cis-12,cis-15-octadécadiénoïque, l'acide 10-hydroxy-cis-6,cis-12-octadécadiénoïque, l'acide 10-hydroxy-cis-6,cis-12,cis-15-octadécatriénoïque, l'acide 10,12-dihydroxy-octadécanoïque, l'acide 10-hydroxy-octadécanoïque, l'acide 10-hydroxy-cis-15-octadécénoïque, l'acide 10-hydroxy-cis-6-octadécénoïque, l'acide 10-hydroxy-cis-6,cis-15-octadécadiénoïque, l'acide 10-hydroxy-trans-11-octadécénoïque, l'acide 10-hydroxy-trans-11,cis-15-octadécadiénoïque, l'acide 10-hydroxy-cis-6,trans-11-octadécadiénoïque, l'acide 10-hydroxy-cis-6,trans-11,cis-15-octadécatriénoique, l'acide 10-oxo-cis-12-octadécénoïque, l'acide 10-oxo-cis-12,cis-15-octadécadiénoïque, l'acide 10-oxo-cis-6,cis-12-octadécadiénoïque, l'acide 10-oxo-cis-6,cis-12,cis-15-octadécatriénoïque, l'acide 10-oxo-octadécanoïque, l'acide 10-oxo-cis-6-octadécénoïque, l'acide 10-oxo-cis-15-octadécénoïque, l'acide 10-oxo-cis-6,cis-15-octadécadiénoïque, l'acide 10-oxo-cis-6,trans-11-octadécadiénoïque, l'acide 10-oxo-trans-11,cis-15-octadécadiénoïque, et l'acide 10-oxo-cis-6,trans-11,cis-15-octadécatriénoïque,
(2) un acide gras saturé ou un acide gras insaturé ayant au moins une double liaison cis en position 6, en position 9, en position 15, qui a 18 atomes de carbone et un groupe hydroxyle ou un groupe carbonyle en position 13, choisi dans l'ensemble constitué par l'acide 13-hydroxy-cis-9-octadécénoïque, l'acide 13-hydroxy-cis-6,cis-9-octadécadiénoïque, l'acide 13-hydroxy-cis-9,cis-15-octadécadiénoïque, l'acide 13-hydroxy-cis-6,cis-9,cis-15-octadécatriénoïque, l'acide 10,13-dihydroxy-octadécanoïque, l'acide 10,13-dihydroxy-cis-6-octadécénoïque, l'acide 10,13-dihydroxy-cis-15-octadécénoïque, l'acide 10,13-dihydroxy-cis-6,cis-15-octadécadiénoïque, l'acide 10-oxo-13-hydroxy-cis-6-octadécénoïque, l'acide 10-oxo-13-hydroxy-cis-15-octadécénoïque, l'acide 10-oxo-13-hydroxy-cis-6,cis-15-octadécadiénoïque, l'acide 13-hydroxy-cis-5,cis-9-octadécadiénoïque, l'acide 13-hydroxy-trans-5,cis-9-octadécadiénoïque, l'acide 13-oxo-cis-9-octadécénoïque, l'acide 13-oxo-cis-6,cis-9-octadécadiénoïque, l'acide 13-oxo-cis-9,cis-15-octadécadiénoïque, l'acide 13-oxo-cis-6,cis-9,cis-15-octadécatriénoïque, l'acide 10,13-dioxo-octadécanoïque, l'acide 10,13-dioxo-cis-6-octadécénoïque, l'acide 10,13-dioxo-cis-15-octadécénoïque, l'acide 10,13-dioxo-cis-6,cis-15-octadécadiénoïque, l'acide 13-oxo-cis-5,cis-9-octadécadiénoïque, et l'acide 13-oxo-trans-5,cis-9-octadécadiénoïque,
(3) un acide gras saturé ou un acide gras insaturé ayant au moins une double liaison cis en position 5, en position 8, en position 9, en position 14, en position 17, qui a 18 ou 20 atomes de carbone et un groupe hydroxyle ou un groupe carbonyle en position 12, choisi dans l'ensemble constitué par l'acide 12-hydroxy-cis-14-éicosénoïque, l'acide 12-hydroxy-cis-14,cis-17-éicosadiénoïque, l'acide 12-hydroxy-cis-8,cis-14-éicosadiénoïque, l'acide 12-hydroxy-cis-5,cis-8-éicosadiénoïque, l'acide 12-hydroxy-cis-8,cis-14,cis-17-éicosatriénoïque, l'acide 12-hydroxy-cis-5,cis-8,cis-14-éicosatriénoïque, l'acide 12-oxo-octadécanoïque, l'acide 12-oxo-cis-14-éicosénoïque, l'acide 12-oxo-cis-14,cis-17-éicosadiénoïque, l'acide 12-oxo-cis-8,cis-14-éicosadiénoïque, l'acide 12-oxo-cis-5,cis-8-éicosadiénoïque, l'acide 12-oxo-cis-8,cis-14,cis-17-éicosatriénoïque, et l'acide 12-oxo-cis-5,cis-8,cis-14-éicosatriénoïque,
(4) un acide gras insaturé ayant au moins une double liaison cis en position 5, en position 8, en position 11, en position 17, qui a 20 atomes de carbone et un groupe hydroxyle ou un groupe carbonyle en position 15, choisi dans l'ensemble constitué par l'acide 15-hydroxy-cis-11-éicosénoïque, l'acide 15-hydroxy-cis-11,cis-17-éicosadiénoïque, l'acide 15-hydroxy-cis-8,cis-11-éicosadiénoïque, l'acide 15-hydroxy-cis-5,cis-8,cis-11-éicosatriénoïque, l'acide 15-hydroxy-cis-8,cis-11,cis-17-éicosatriénoïque, l'acide 15-hydroxy-cis-5,cis-11-éicosadiénoïque, l'acide 15-hydroxy-cis-5,cis-11,cis-17-éicosatriénoïque, l'acide 15-oxo-cis-11-éicosénoïque, l'acide 15-oxo-cis-11,cis-17-éicosadiénoïque, l'acide 15-oxo-cis-8,cis-11-éicosadiénoïque, l'acide 15-oxo-cis-5,cis-8,cis-11-éicosatriénoïque, l'acide 15-oxo-cis-8,cis-11,cis-17-éicosatriénoïque, l'acide 15-oxo-cis-5,cis-11-éicosadiénoïque, et l'acide 15-oxo-cis-5,cis-11,cis-17-éicosatriénoïque, ou
(5) un acide gras saturé ayant 16 atomes de carbone et un groupe hydroxyle ou un groupe carbonyle en position 10, choisi dans l'ensemble constitué par l'acide 10-hydroxy-hexadécanoïque et l'acide 10-oxo-hexadécanoïque,
pour une utilisation pour la prophylaxie ou le traitement d'une maladie inflammatoire.

2. Agent pour une utilisation selon la revendication 1, comprenant l'acide gras suivant :
(1) un acide gras saturé ou un acide gras insaturé ayant une double liaison trans en position 11 ou au moins une double liaison cis en position 6, en position 12, qui a 18 atomes de carbone et un groupe hydroxyle ou un groupe carbonyle en position 10, choisi dans l'ensemble constitué par l'acide 10-hydroxy-cis-12-octadécénoïque, l'acide 10-hydroxy-octadécanoïque, l'acide 10-oxo-cis-12-octadécénoïque, l'acide 10-hydroxy-cis-12,cis-15-octadécadiénoïque, l'acide 10-hydroxy-cis-6,cis-12-octadécadiénoïque, l'acide 10-oxo-cis-12,cis-15-octadécadiénoïque, l'acide 10-oxo-cis-6,cis-12-octadécadiénoïque, l'acide 10-oxo-trans-11,cis-15-octadécadiénoique, et l'acide 10-oxo-cis-6,trans-11-octadécadiénoïque, ou
(2) un acide gras saturé ou un acide gras insaturé ayant au moins une double liaison cis en position 6, en position 9, en position 15, qui a 18 atomes de carbone et un groupe hydroxyle ou un groupe carbonyle en position 13, choisi dans l'ensemble constitué par l'acide 13-hydroxy-cis-9-octadécénoïque, l'acide 13-hydroxy-cis-6,cis-9-octadécadiénoïque, l'acide 13-hydroxy-cis-9,cis-15-octadécadiénoïque, l'acide 10,13-dihydroxy-octadécanoïque, l'acide 10,13-dihydroxy-cis-6-octadécénoïque, l'acide 10,13-dihydroxy-cis-15-octadécénoïque, l'acide 13-oxo-cis-9-octadécénoïque, l'acide 13-oxo-cis-6,cis-9-octadécadiénoique, et l'acide 13-oxo-cis-9,cis-15-octadécadiénoïque.

3. Agent pour une utilisation selon la revendication 1 ou 2, pour une utilisation pour la prophylaxie ou l'amélioration d'une maladie inflammatoire impliquant un macrophage.

4. Agent pour une utilisation selon la revendication 1 ou 2, lequel agent est un aliment ou un additif alimentaire.

5. Agent pour une utilisation selon la revendication 1 ou 2, lequel agent est un produit pharmaceutique.

6. Agent pour une utilisation selon la revendication 1 ou 2, lequel agent est un aliment pour animaux ou un additif pour aliment pour animaux.
